# EUROPEAN PATENT SPECIFICATION

(11) **EP 1 156 787 B1**
(45) Date of publication and mention of the grant of the patent: **30.11.2005**
(21) Application number: 00913480.0
(22) Date of filing: 11.02.2000
(51) Int. Cl.: C07D 271/10, A61K 47/22, A61K 9/20, A61K 9/48, A61K 31/415

(54) **OXADIAZOLE COMPOUNDS AND COMPOSITIONS FOR DELIVERING ACTIVE AGENTS**
OXADIAZOLVERBINDUNGEN UND -ZUSAMMENSETZUNGEN ZUR ABGABE VON WIRKSTOFFEN
COMPOSES A BASE D'OXADIAZOLE ET COMPOSITIONS DESTINEES A L'APPORT D'AGENTS ACTIFS

(30) Priority: 11.02.1999 US 119638 P
(43) Date of publication of application: 28.11.2001
(73) Proprietor: Emisphere Technologies, Inc., Tarrytown, New York 10591 (US)
(72) Inventor: GSCHNEIDNER, David, Stamford, CT 06907 (US)
(74) Representative: Grünecker, Kinkeldey, Stockmair & Schwanhäusser Anwaltssozietät
(86) International application number: PCT/US2000/003899
(87) International publication number: WO 2000/047188

(56) References cited:
- EP-A- 0 608 858
- WO-A-95/28920
- DE-A- 2 651 386
- JP-B- 42 020 057
- US-A- 2 765 304
- US-A- 3 964 896
- DATABASE CROSSFIRE BEILSTEIN [Online] Beilstein Institut zur Förderung der Chemischen Wissenschaften, Frankfurt am Main, DE; Database accession no. (Beilstein Registry Number) 6726717 XP002190634 & J. INDIAN CHEM. SOC., vol. 65, 1988, pages 521-522,
- DATABASE CROSSFIRE BEILSTEIN [Online] Beilstein Institut zur Förderung der Chemischen Wissenschaften, Frankfurt am Main, DE; Database accession no. (Beilstein Registry Number) 522764 XP002190635 & CAN. J. CHEM., vol. 46, 1968, pages 2255-2261,
- MAILLARD J ET AL: "Anti-inflammatoires dérivés de l'acide phénylacétique; dérivés substitués par un hétérocycle sur le noyau phényle" CHIMIE THERAPEUTIQUE, vol. 8, 1 July 1973 (1973-07-01), pages 487-494, XP002079843
- TAKAHASHI M ET AL: "The synthesis of 1,3,4-oxadiazolo[3,2-b]isoquinoline and 1,3,4-thiadiazolo[3,2-b]isoquinoline derivatives from homopthalic anhydride" BULLETIN OF THE CHEMICAL SOCIETY OF JAPAN, vol. 48, no. 10, October 1975 (1975-10), pages 2915-2917, XP002190631
- MICETICH R G ET AL: "Antibacterial activity of 6-(5-membered heteroarylacetamido)penicillanic acids" JOURNAL OF MEDICINAL CHEMISTRY, vol. 15, no. 3, March 1972 (1972-03), pages 333-335, XP002190632
- SHORT F W ET AL: "Synthesis of 5-aryl-2-oxazolepropionic acids and analogs. Antiinflamatory agents" JOURNAL OF HETROCYCLIC CHEMISTRY, vol. 6, no. 5, October 1969 (1969-10), pages 707-712, XP002190633
- ANDOTRA, C. S.: 'Synthesis and biocidal activity of some 1,3,4-oxadiazolo(3,2-a)-s-triazine-5-7-dith iones' INDIAN J. HETEROCYCL. CHEM. vol. 1, no. 1, June 1991, pages 26 - 28, XP002928979
- KAHN ET. AL.: 'Insulin inhibits vascular smooth muscle contraction at a site distal to intracellular ca2 + concentration' AM. J. PHYSIOL vol. 274, no. 37, 1998, pages E885 - E892, XP002928980
- HASDAI ET. AL.: 'Insulin and Insulin-like Growth Factor-I Cause Coronary Vasorelaxation in Vitro' HYPERTENSION vol. 32, August 1998, pages 228 - 234, XP002928981

## Description

The present invention relates to oxadiazole compounds for delivering active agents, such as biologically or chemically active agents, to a target. These compounds are well suited for forming non-covalent mixtures with active agents for oral, intracolonic, or other routes of administration to animals. Methods for the preparation and administration of such compositions are also disclosed.

Conventional means for delivering active agents are often severely limited by biological, chemical, and physical barriers. Typically, these barriers are imposed by the environment through which delivery occurs, the environment of the target for delivery, and/or the target itself. Biologically and chemically active agents are particularly vulnerable to such barriers.

In the delivery to animals of biologically active and chemically active pharmacological and therapeutic agents, barriers are imposed by the body. Examples of physical barriers are the skin, lipid bi-layers and various organ membranes that are relatively impermeable to certain active agents but must be traversed before reaching a target, such as the circulatory system. Chemical barriers include, but are not limited to, pH variations in the gastrointestinal (GI) tract and degrading enzymes.

These barriers are of particular significance in the design of oral delivery systems. Oral delivery of many biologically or chemically active agents would be the route of choice for administration to animals if not for biological, chemical, and physical barriers. Among the numerous agents which are not typically amenable to oral administration are biologically or chemically active peptides, such as calcitonin and insulin; polysaccharides, and in particular mucopolysaccharides including, but not limited to, heparin; heparinoids; antibiotics; and other organic substances. These agents may be rapidly rendered ineffective or destroyed in the gastro-intestinal tract by acid hydrolysis, enzymes, and the like. In addition, the size and structure of macromolecular drugs may prohibit absorption.

Earlier methods for orally administering vulnerable pharmacological agents have relied on the co-administration of adjuvants (e.g., resorcinols and non-ionic surfactants such as polyoxyethylene oleyl ether and n-hexadecylpolyethylene ether) to increase artificially the permeability of the intestinal walls, as well as the co-administration of enzymatic inhibitors (e.g., pancreatic trypsin inhibitors, diisopropylfluorophosphate (DFF) and trasylol) to inhibit enzymatic degradation. Liposomes have also been described as drug delivery systems for insulin and heparin. However, broad spectrum use of such drug delivery systems is precluded because: (1) the systems require toxic amounts of adjuvants or inhibitors; (2) suitable low molecular weight cargos, i.e. active agents, are not available; (3) the systems exhibit poor stability and inadequate shelf life; (4) the systems are difficult to manufacture; (5) the systems fail to protect the active agent (cargo); (6) the systems adversely alter the active agent; or (7) the systems fail to allow or promote absorption of the active agent.

More recently, proteinoid microspheres have been used to deliver pharmaceuticals. For example, see US 5,401,516, US 5,443,841 and US RE35,862. In addition, certain modified amino acids have been used to deliver pharmaceuticals. See, e.g., US 5,629,020; US 5, 643, 957; US 5, 766, 633; US 5,776,888; and US 5,866,536.

WO 95/28920 discloses a delivery system and in particular carboxylic acids such as phenyl propanoic acid for use as a delivery system of sensitive agents such as bioactive peptides.

The problem underlying the present invention is to provide simple, inexpensive delivery systems which are easily prepared and which can deliver a broad range of active agents by various routes.

The problem is solved by a compound of the formula 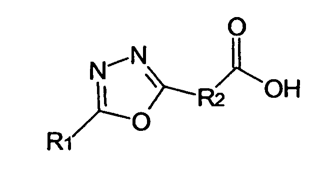 or a salt thereof, wherein
R¹ is C₃-C₁₀ cycloalkyl or naphthyl; optionally substituted with C₁-C₄ alkyl or fluoroalkyl, C₂-C₄ alkenyl, C₁-C₄ alkoxy or fluoroalkoxy, halogens, -OH, -SH, phenyl, phenoxy, -CO₂R³, -N(CH₃)₂, -NO₂, or -NH₂;
R¹ further is 2-hydroxy phenol; optionally substituted with -OH, -Cl, -CH₃ or -OCH₃;
R² is C₁-C₂₄ alkylene, C₂-C₂₄ alkenylene, C₃-C₁₀ cycloalkylene, C₃-C₁₀ cycloalkenylene, phenylene, naphthylene, (C₁-C₁₀ alkyl)phenylene, (C₂-C₁₀ alkenyl)phenylene, (C₁-C₁₀ alkyl)naphthylene, (C₂-C₁₀ alkenyl)naphthylene, phenyl(C₁-C₁₀ alkylene), phenyl(C₂-C₁₀ alkenylene), naphthyl(C₁-C₁₀ alkylene) or naphthyl(C₂-C₁₀ alkenylene);
R² is optionally substituted with C₁-C₄ alkyl or fluoroalkyl, C₂-C₄ alkenyl, C₁-C₄ alkoxy or fluoroalkoxy, halogens, -OH, -SH, phenyl, phenoxy, -CO₂R³, -N(CH₃)₂, -NO₂, -NH₂, C₃-C₁₀ cycloalkyl, C₃-C₁₀ cycloalkenyl, aryl, (C₁-C₁₀ alk)aryl, heterocycle having 3-10 ring atoms wherein the hetero atom is one or more of N, O, S or any combination thereof, or any combination thereof;
R² being optionally interrupted by oxygen, nitrogen, sulfur, or any combination thereof; and
R³ is hydrogen, C₁-C₄ alkyl, or C₂-C₄ alkenyl.

Preferred embodiments are set forth in the subclaims. Further embodiments are set forth in the claims.

Preferably, if applicable, R¹ is C₁-C₆ alkyl, C₂-C₆ alkenyl, C₂-C₆ alkynyl, cycloalkyl, phenyl, or naphthyl and R¹ is optionally substituted with halogens, -OH, -SH, C₁-C₄ alkyl or C₁-C₄ alkoxy. More preferably, R¹ is a substituted or unsubstituted phenyl or naphthyl, -CH₃, or -CH₂OH. Most preferably, R¹ is 2-OH-phenyl, optionally further substituted with -OH, Cl, -CH₃ or -OCH₃.

Preferably, R² is C₁-C₂₄ alkylene or (C₁-C₁₀ alkyl)phenylene. More preferably, R² is C₄-C₈ alkylene.

More preferred compounds include, but are not limited to, those having the formulas: 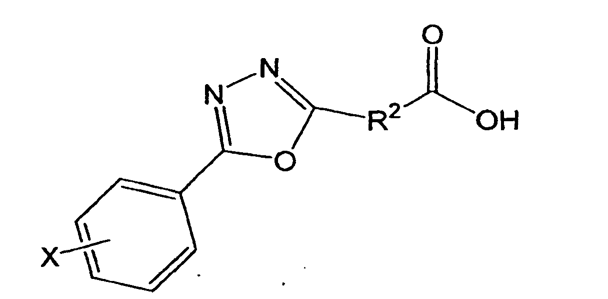 wherein

| **Cpd #** | **X** | **R**^{**2**} |
|---|---|---|
| | | |
| 1 | 2-OH | (CH₂) ₈ |
| 2 | 2-OH | (CH₂) ₆ |
| 3 | 2-OH | (CH₂) ₄ |
| 4 | 2-OH | (CH₂) ₇ |
| 5 | 2-OH-5-Cl | (CH₂) ₆ |
| 6 | 2-OH-3,5-di-Cl | (CH₂)₆ |
| 7 | 2-OH-4-methyl | (CH₂) ₆ |
| 8 | 2-OH-4-OMe | (CH₂) ₆ |
| 9 | 2-OH-4-OMe | (CH₂) ₄ |
| 10 | H | (CH₂)₆ |
| 11 | 2-Cl | (CH₂) ₆ |

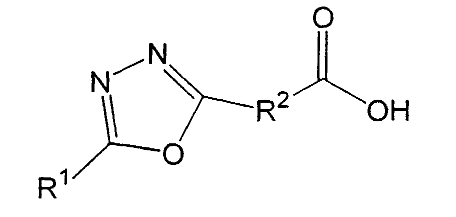

wherein

| **Cpd#** | **R**^{**1**} | **R**^{**2**} |
|---|---|---|
| | | |
| 12 | CH₃ | (CH₂) ₆ |
| 13 | CH₂OH | (CH₂) ₆ |

polymorphs thereof, and salts thereof.

These compounds may be referred to as oxadiazoles.

The compositions of the present invention comprise at least one active agent, preferably a biologically or chemically active agent, and at least one of the compounds or salts thereof as set forth in the claims, including all the particular embodiments referred to above, and also including compounds 1-13. These compounds may have the formula 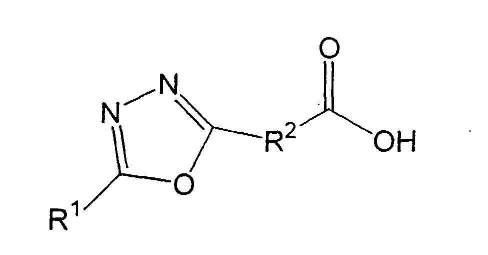 wherein
R¹ is C₁-C₁₀ alkyl, C₂-C₁₀ alkenyl, C₂-C₁₀ alkynyl, C₃-C₁₀ cycloalkyl, phenyl, naphthyl, or aromatic heterocycle;
R² is optionally substituted with C₁-C₄ alkyl or fluoroalkyl, C₂-C₄ alkenyl, C₁-C₄ alkoxy or fluoroalkoxy, halogens, -OH, -SH, phenyl, phenoxy, -CO₂R₃, -N(CH₃)₂, -NO₂, or -NH₂.
R² is C₁-C₂₄ alkylene, C₂-C₂₄ alkenylene, C₃-C₁₀ cycloalkylene, C₃-C₁₀ cycloalkenylene, phenylene, naphthylene, (C₁-C₁₀ alkyl)phenylene, (C₂-C₁₀ alkenyl)phenylene, (C₁-C₁₀ alkyl)naphthylene, (C₂-C₁₀ alkenyl)naphthylene, phenyl (C₁-C₁₀ alkylene), phenyl (C₂-C₁₀ alkenylene), naphthyl (C₁-C₁₀ alkylene) or naphthyl (C₂-C₁₀ alkenylene);
R² is optionally substituted with C₁-C₄ alkyl or fluoroalkyl, C₂-C₄ alkenyl, C₁-C₄ alkoxy or fluoroalkoxy, halogens, -OH, -SH, phenyl, phenoxy, -CO₂R₃, -N(CH₃)₂, -NO₂, - NH₂, C₃-C₁₀ cycloalkyl, C₃-C₁₀ cycloalkenyl, aryl, (C₁-C₁₀ alk)aryl, heterocycle having 3-10 ring atoms wherein the hetero atom is one or more of N, 0, S or any combination thereof, or any combination thereof;
R² being optionally interrupted by oxygen, nitrogen, sulfur, or any combination thereof; and
R³ is hydrogen, C₁-C₄ alkyl, or C₂-C₄ alkenyl. Methods for the preparation and administration of such compositions are also provided.

Also provided are dosage unit forms comprising the composition of the present invention. The dosing vehicle can be a solid (such as a tablet, powder, or capsule) or a liquid.

Methods for administering a biologically active agent to an animal in need of the agent, especially by the oral or intracolonic routes, with the compositions of the present invention, are also provided.

### Compounds

The compounds may be in the form of the carboxylic acid and/or their salts. Salts include but are not limited to organic and inorganic salts, for example alkali-metal salts, such as sodium, potassium and lithium; alkaline-earth metal salts, such as magnesium, calcium or barium; ammonium salts; basic amino acids, such as lysine or arginine; and organic amines, such as dimethylamine or pyridine. Preferably, the salts are sodium salts.

In general, the compounds of the present invention may be prepared by dehydrative cyclization of dihydrazides, protecting any functional groups present in the dihydrazide as necessary. Dehydrative cyclization may be performed by reacting dihydrazide with carbon tetrachloride and triphenylphosphene in the presence of an organic base, such as triethylamine. The acid is formed from basic hydrolysis of the resulting ester. Suitable solvents for the cyclization reaction include, but are not limited to, acetonitrile.

The compound may be purified by recrystallization or by fractionation on one or more solid chromatographic supports, alone or linked in tandem. Suitable recrystallization solvent systems include, but are not limited to, acetonitrile, methanol, and tetrahydrofuran. Fractionation may be performed on a suitable chromatographic support such as alumina, using methanol/n-propanol mixtures as the mobile phase; reverse phase chromatography using trifluoroacetic acid/acetonitrile mixtures as the mobile phase; and ion exchange chromatography using water or an appropriate buffer as the mobile phase. When anion exchange chromatography is performed, preferably a 0-500 mM sodium chloride gradient is employed.

### Active Agents

Active agents suitable for use in the present invention include biologically active agents and chemically active agents, including, but not limited to, pesticides, pharmacological agents, and therapeutic agents.

For example, biologically or chemically active agents suitable for use in the present invention include, but are not limited to, proteins; polypeptides; peptides; hormones; polysaccharides, and particularly mixtures of mucopolysaccharides; carbohydrates; lipids; other organic compounds; and particularly compounds which by themselves do not pass (or which pass only a fraction of the administered dose) through the gastro-intestinal mucosa and/or are susceptible to chemical cleavage by acids and enzymes in the gastro-intestinal tract; or any combination thereof.

Further examples include, but are not limited to, the following, including synthetic, natural or recombinant sources thereof: growth hormones, including human growth hormones (hGH), recombinant human growth hormones (rhGH), bovine growth hormones, and porcine growth hormones; growth hormone-releasing hormones; interferons, including α, β and γ; interleukin-1; interleukin-2; insulin, including porcine, bovine, human, and human recombinant, optionally having counter ions including sodium, zinc, calcium and ammonium; insulin-like growth factor, including IGF-1; heparin, including unfractionated heparin, heparinoids, dermatans, chondroitins, low molecular weight heparin, very low molecular weight heparin and ultra low molecular weight heparin; calcitonin, including salmon, eel and human; erythropoietin; atrial naturetic factor; antigens; monoclonal antibodies; somatostatin; protease inhibitors; adrenocorticotropin, gonadotropin releasing hormone; oxytocin; leutinizing-hormone-releasing-hormone; follicle stimulating hormone; glucocerebrosidase; thrombopoietin; filgrastim; prostaglandins; cyclosporin; vasopressin; cromolyn sodium (sodium or disodium chromoglycate); vancomycin; desferrioxamine (DFO); parathyroid hormone (PTH), including its fragments; antimicrobials, including anti-fungal agents; vitamins; analogs, fragments, mimetics or polyethylene glycol (PEG)-modified derivatives of these compounds; or any combination thereof.

### Delivery systems

The compositions of the present invention comprise a delivery agent, i.e., a compound of the present invention, and one or more active agents. In one embodiment, one or more of the delivery agent compounds, or salts of these compounds, may be used as a delivery agent by mixing with the active agent prior to administration.

The administration compositions may be in the form of a liquid. The solution medium may be water (for example, for salmon calicitonin, parathyroid hormone, and erthyropoietin), 25% aqueous propylene glycol (for example, for heparin) and phosphate buffer (for example, for rhGH). Other dosing vehicles include polyethylene glycols, sorbitol, maltitol, and sucrose. Dosing solutions may be prepared by mixing a solution of the delivery agent compound with a solution of the active agent, just prior to administration. Alternately, a solution of the delivery agent (or active agent) may be mixed with the solid form of the active agent (or delivery agent). The delivery agent compound and the active agent may also be mixed as dry powders. The delivery agent compound and the active agent can also be admixed during the manufacturing process.

The dosing solutions may optionally contain additives such as phosphate buffer salts, citric acid, glycols, or other dispersing agents. Stabilizing additives may be incorporated into the solution, preferably at a concentration ranging between about 0.1 and 20% (w/v).

The administration compositions may alternately be in the form of a solid, such as a tablet, capsule or powder. Solid dosage forms may be prepared by mixing the solid form of the compound with the solid form of the active agent.
Alternately, a solid may be obtained from a solution of compound and active agent by methods known in the art, such as freeze drying, precipitation, crystallization and solid dispersion.

The administration compositions of the present invention may also include one or more enzyme inhibitors. Such enzyme inhibitors include, but are not limited to, compounds such as actinonin, epiactinonin, and derivatives thereof. Other enzyme inhibitors include, but are not limited to, aprotinin (Trasylol) and Bowman-Birk inhibitor.

The amount of active agent used in an administration composition of the present invention is an amount effective to accomplish the purpose of the particular active agent for the target indication. The amount of active agent in the compositions typically is a pharmacologically, biologically, therapeutically, or chemically effective amount. However, the amount can be less than that amount when the composition is used in a dosage unit form because the dosage unit form may contain a plurality of compound/active agent compositions or may contain a divided pharmacologically, biologically, therapeutically, or chemically effective amount. The total effective amount can then be administered in cumulative units containing, in total, an effective amount of the active agent.

The total amount of active agent to be used can be determined by methods known to those skilled in the art. However, because the compositions may deliver active agents more efficiently than prior compositions, lower amounts of biologically or chemically active agents than those used in prior dosage unit forms or delivery systems can be administered to the subject, while still achieving the same blood levels and/or therapeutic effects.

The presently disclosed compounds facilitate delivery of biologically and chemically active agents, particularly in oral, intranasal, sublingual, intraduodenal, subcutaneous, buccal, intracolonic, rectal, vaginal, mucosal, pulmonary, transdermal, intradermal, parenteral, intravenous, intramuscular and ocular systems, as well as traversing the blood-brain barrier.

Dosage unit forms can also include any one or combination of excipients, diluents, disintegrants, lubricants, plasticizers, colorants, flavorants, taste-masking agents, sugars, sweeteners, salts, and dosing vehicles, including, but not limited to, water, 1,2-propane diol, ethanol, olive oil, or any combination thereof.

The compounds and compositions of the subject invention are useful for administering biologically or chemically active agents to any animals, including but not limited to birds such as chickens; mammals, such as rodents, cows, pigs, dogs, cats, primates, and particularly humans; and insects.

The present invention is particularly advantageous for delivering chemically or biologically active agents that would otherwise be destroyed or rendered less effective by conditions encountered before the active agent reaches its target zone (i.e. the area in which the active agent of the delivery composition is to be released) and within the body of the animal to which they are administered. Particularly, the compounds and compositions of the present invention are useful in orally administering active agents, especially those that are not ordinarily orally deliverable, or those for which improved delivery is desired.

The compositions comprising the compounds and active agents have utility in the delivery of active agents to selected biological systems and in an increased or improved bioavailability of the active agent compared to administration of the active agent without the delivery agent. Delivery can be improved by delivering more active agent over a period of time, or in delivering active agent in a particular time period (such as to effect quicker or delayed delivery) or over a period of time (such as sustained delivery).

Following administration, the active agent present in the composition or dosage unit form is taken up into the circulation. The bioavailability of the agent is readily assessed by measuring a known pharmacological activity in blood, e.g. an increase in blood clotting time caused by heparin, or a decrease in circulating calcium levels caused by calcitonin. Alternately, the circulating levels of the active agent itself can be measured directly.

The following examples illustrate the invention without limitation. All parts are given by weight unless otherwise indicated.

### Example 1

Compound 2 was prepared as follows. A suspension of 30.34 g (0.108 mol) of N-(methyl suberoyl)-N'-salicyloylhydrazide and 138 mL of methylene chloride was cooled to 0°C in an ice bath and treated with 16.56 mL (12.0 g, 0.119 mol) of triethylamine, followed by 14.40 mL (12.3 g, 0.113 mol) of trimethylsilylchloride 15 minutes later. After warming to 25°C and stirring for 3 hours, the reaction mixture was concentrated in vacuo.

The residue was taken up in 183 mL of acetonitrile and treated with 45.16 mL (32.8 g, 0.324 mol) of triethylamine, 50.4 mL (80.2 g, 0.522 mol) of carbon tetrachloride and 63.88 g (0.243 mol) of triphenylphosphine. The orange slurry was stirred for 18 hours. The solid was removed by filtration. The filtrate was diluted with 183 mL of ethyl acetate, washed with 2% aqueous hydrochloric acid (2x100 mL) dried over sodium sulfate and concentrated in vacuo.

The resulting solid was brought up in 60 mL of 2N aqueous sodium hydroxide and stirred at 65 °C for three hours. The undissolved solid was removed by filtration. The filtrate was acidified to pH 3 with 3% aqueous hydrochloric acid. The resulting solid was isolated by filtration and recrystallized from ethanol/water. A total of 11.76 g, having a melting point of 109-112 °C, was collected.

Compounds 1, 3-8, 10 and 12 were also prepared by this method with the appropriate starting materials. Compounds 9, 11 and 13 may also be prepared by this method using the appropriate starting materials.

### Example 2 - Salmon Calcitonin (sCT)

### Oral Delivery

Oral dosing (PO) compositions of delivery agent compound and salmon calcitonin (sCT) in water were prepared. Typically, 450 mg of compound was added to 2.0 ml of water. Either the sodium salt of the compound was used or the free acid was converted to the sodium salt by stirring the resultant solution and adding one equivalent of sodium hydroxide (1.0 N) and diluting with water. The solution was vortexed, then heated (about 37°C) and sonicated. The pH was adjusted to about 7 (6.5 to 8.5) with sodium hydroxide or hydrochloric acid. 90 µg sCT from a stock solution was added to the solution. Water was then added to bring the total volume to about 3.0 ml (varies depending on solubility of the delivery agent compound) and vortexed. The final delivery agent compound dose, sCT dose and volume dose amounts are listed below in Table 1.

The typical dosing and sampling protocols were as follows. Male Sprague-Dawley rats weighing between 200-250g were fasted for 24 hours and administered ketamine (44 mg/kg) and chlorpromazine (1.5 mg/kg) 15 minutes prior to dosing. A dosing group of five rats was administered one of the dosing solutions. For oral dosing, an 11 cm Rusch 8 French catheter was adapted to a 1 ml syringe with a pipette tip. The syringe was filled with dosing solution by drawing the solution through the catheter, which was then wiped dry. The catheter was placed down the esophagus leaving 1 cm of tubing past the incisors. Solution was administered by pressing the syringe plunger.

Blood samples were collected serially from the tail artery, typically at 0, 10, 20, 30, 60 and 90 minutes after administration. Serum sCT was determined by testing with an EIA kit (Kit # EIAS-6003 from Peninsula Laboratories, Inc., San Carlos, CA). Numbers were adjusted according to baseline values obtained at 0 minutes. The limit of the assay is 80 pg/ml. The results from the five rats in each dosing group were averaged for each time point. The maximum is reported below in Table 1.

**Table 1**

| Oral Delivery of Salmon Calcitonin (sCT) | | | | |
|---|---|---|---|---|
| Compound | Volume Dose (ml/kg) | Compound Dose (mg/kg) | sCT Dose (µg/kg) | Mean Peak Serum Set (pg/ml ± SD)(SE) |
| 1 | 1 | 150 | 30 | 167 ± 89 (39) |
| 2 | 1 | 150 | 30 | 260 ± 146 (65) |
| 2 | 1 | 150 | 30 | 112 ± 69 (31) |
| 2 | 1 | 150 | 30 | 165 ± 136 (61) |
| 2 | 1 | 150 | 30 | 160 ± 207 (93) |
| 2 | 1 | 150 | 30 | 492 ± 830 (10) |
| *3 | 1 | 150 | 30 | 271 ± 503 (225) |
| 3 | 1 | 150 | 30 | 444 ± 277 (124) |
| 3 | 1 | 150 | 30 | 108 ± 115 (52) |
| 4 | 1 | 150 | 30 | 272 ± 284 (127) |
| 4 | 1 | 150 | 30 | 253 ± 374 (167) |
| 5 | 1 | 150 | 30 | 393 ± 431 (193) |
| 6 | 1 | 150 | 30 | 306 ± 339 (152) |
| 8 | 1 | 150 | 30 | 168 ± 217 (97) |
| 8 | 1 | 150 | 30 | 204 ± 227 (102) |
| 10 | 1 | 150 | 30 | 37 ± 55 |
| 12 | 1 | 150 | 30 | 212 ± 194 (433) |
| 12 | 1 | 150 | 30 | 155 ± 134 (60) |

| | | | | |
|---|---|---|---|---|
| * In this dosing of compound 3, the standard protocol was modified from the kit as follows: incubated with 50 µl peptide antibody for 2 hours with shaking in the dark, washed the plate, added serum and biotinylated peptide and diluted with 4 ml buffer, and shook overnight in the dark. | | | | |

### Example 3 - Heparin Delivery

### Oral/Intracolonic Delivery

Oral gavage (PO) and/or intracolonic (IC) dosing solutions containing a delivery agent compound and heparin sodium USP in 25% aqueous propylene glycol were prepared. Either the sodium salt of the compound was used or the free acid was converted to the sodium salt with one equivalent of sodium hydroxide. Typically, delivery agent compound and heparin (about 166-182 IU/mg) were mixed by vortex as dry powders. This dry mixture was dissolved in 25% v/v aqueous propylene glycol, vortexed and placed in a sonicator (about 37 °C). The pH was adjusted to about 7 (6.5 to 8.5) with aqueous sodium hydroxide (2N). The dosing solution was sonicated to produce a clear solution. The final volume was adjusted to about 3.0 ml. The final delivery agent compound dose, heparin dose and volume dose amounts are listed below in Table 2.

The typical dosing and sampling protocols were as follows. Male Sprague-Dawley rats weighing between 275-350g were fasted for 24 hours and were anesthetized with ketamine hydrochloride (88 mg/kg) intramuscularly immediately prior to dosing. A dosing group of five rats was administered one of the dosing solutions. For oral gavage (PO) dosing, an 11cm Rusch 8 French catheter was adapted to a 1 ml syringe with a pipette tip. The syringe was filled with dosing solution by drawing the solution through the catheter, which was then wiped dry. The catheter was placed down the esophagus leaving 1 cm of tubing past the rat's incisors. Solution was administered by pressing the syringe plunger. For intracolonic (IC) dosing, a 7.5cm, 8 fr Rusch catheter was adapted to a 1 ml syringe with a pipette tip. The dosing catheter was inserted into the colon through the anus until the tube was no longer visible. The dosing solution was expressed slowly into the colon by pressing the syringe plunger.

Citrated blood samples were collected by cardiac puncture following the administration of ketamine (88 mg/kg), typically at 0.25, 0.5, 1.0 and 1.5 hours after administration. Heparin activity was determined by utilizing the activated partial thromboplastin time (APTT) according to the method of Henry, J.B., Clinical Diagnosis and Management by Laboratory Methods, Philadelphia, PA, W.B. Saunders (1979). Previous studies indicated baseline values of about 20 sec. Results from the five rats in each group were averaged for each time point. The maximum is reported below in Table 2.

**Table 2**

| Oral/Intracolonic Delivery of Heparin | | | | | |
|---|---|---|---|---|---|
| Compound | Method of Administration | Volume dose (ml/kg) | Compound Dose (mg/kg) | Heparin Dose (mg/kg) | Mean Peak APTT (sec) ± SD |
| 1 | IC | 1 | 50 | 25 | 38.4 ± 17.4 |
| 2 | IC | 1 | 50 | 25 | 52.8 ± 31.9 |

### Example 4 - Recombinant Human Growth Hormone (rhGH) Oral/Intracolonic Delivery

Oral gavage (PO) and/or intracolonic (IC) dosing solutions of delivery agent compound and rhGH in phosphate buffer were prepared. A solution of the compound was made either with the sodium salt of the compound or by converting the free acid to its sodium salt. Typically, a solution of the compound was prepared in phosphate buffer and stirred, adding one equivalent of sodium hydroxide (1.0 N) when making the sodium salt. The final dosing solutions were prepared by mixing the compound solution with an rhGH stock solution (15 mg rhGH/ml) and diluting to the desired volume (usually 3.0 ml). The compounds and rhGH dose amounts are listed below in Table 3.

The typical dosing and sampling protocols were as follows. Male Sprague-Dawley rats weighing between 200-250g were fasted for 24 hours and administered ketamine (44 mg/kg) and chlorpromazine (1.5 mg/kg) 15 minutes prior to dosing. A dosing group of five rats was administered one of the dosing solutions. For oral gavage (PO), an 11cm Rusch 8 French catheter was adapted to a 1 ml syringe with a pipette tip. The syringe was filled with dosing solution by drawing the solution through the catheter, which was then wiped dry. The catheter was placed down the esophagus leaving 1 cm of tubing past the rat's incisors. Solution was administered by pressing the syringe plunger. For intracolonic (IC) dosing, a 7.5 cm Rusch catheter tube (French 8 or 6) was adapted to a syringe with an Eppendorf pipette tip. The syringe was filled with the dosing solution by drawing the solution through the catheter tube. The catheter tube was wiped dry. K-Y jelly was applied to the tip, avoiding contact with the eye of the tube, and the tube was inserted into the colon through the anus until the tube was no longer visible. The solution was injected by pressing the syringe plunger, and the tube was removed.

Blood samples were collected serially from the tail artery, typically at 0, 15, 30, 45, 60 and 90 minutes after oral administration and 0, 10, 20, 30, 60 and 90 minutes after IC administration. The five samples from each time period were pooled (except for where compound 1 was dosed). Serum rHGH concentrations were quantified by an rHGH immunoassay test kit (Kit # K1F4015 from Genzyme Corporation Inc., Cambridge, MA). Previous studies indicated baseline values of about zero.

The maximum concentration for each group and the area under the curve (AUC), when available, are reported below in Table 3.

**Table 3**

| Oral/Intracolonic Delivery of rhGH in Rats | | | | | | |
|---|---|---|---|---|---|---|
| Compound | Method of Administration | Volume dose (ml/kg) | Compound Dose (mg/kg) | rhGH Dose (mg/kg) | Mean Peak Serum [rhGH] (ng/ml) | AUC |
| 1 | PO | 1 | 200 | 3 | 4.42 ± 4.42 | 142.25 |
| 1 | PO | 1 | 200 | 3 | 11.1 ± 8.9 | 218.7 |
| 2 | PO | 1 | 200 | 3 | 3.77 | 56.55 |
| 3 | PO | 1 | 200 | 3 | 88.9 | 133.5 |
| 3 | PO | 1 | 200 | 3 | 35.08 | 969.6 |
| 5 | PO | 1 | 200 | 3 | - 14.64 | 219.6 |
| 6 | PO | 1 | 200 | 3 | 0 | 0 |
| 7 | PO | 1 | 200 | 3 | 0.045 | 60 |
| 8 | PO | 1 | 200 | 3 | 9.81 | 2187.7 |
| 8 | PO | 1 | 200 | 3 | 0 | 0 |
| 9 | PO | 1 | 200 | 3 | 14.25 | |
| 10 | PO | 1 | 200 | 3 | 14.25 | 802.725 |
| 12 | PO | 1 | 200 | 3 | 0 | 0 |

### Example 5 - Parathyroid Hormone Delivery (PTH 1-34) Oral/Intracolonic Delivery

Oral gavage (PO) and/or intracolonic (IC) dosing solutions of delivery agent compound and human parathyroid hormone residues 1-34 (PTH) in water were prepared. A solution of the compound was made either with the sodium salt of the compound or by converting the free acid to its sodium salt. Typically, a solution of the compound was prepared in water and stirred, adding one equivalent of sodium hydroxide (1.0 N) when making the sodium salt. The final dosing solutions were prepared by mixing the compound solution with a PTH stock solution (typically having a concentration of 5 mg PTH/ml) and diluting to the desired volume (usually 3.0 ml). The final compound, PTH and volume dose amounts are listed below in Table 4.

The typical dosing and sampling protocols were as follows. Male Sprague-Dawley rats weighing between 200-250g were fasted for 24 hours and administered ketamine (44 mg/kg) and chlorpromazine (1.5 mg/kg) 15 minutes prior to dosing. A dosing group of five rats was administered one of the dosing solutions. For oral gavage (PO), an 11cm Rusch 8 French catheter was adapted to a 1 ml syringe with a pipette tip. The syringe was filled with dosing solution by drawing the solution through the catheter, which was then wiped dry. The catheter was placed down the esophagus leaving 1 cm of tubing past the rat's incisors. Solution was administered by pressing the syringe plunger. For intracolonic (IC) dosing, a 7.5 cm Rusch catheter tube (French 8 or 6) was adapted to a syringe with an Eppendorf pipette tip. The syringe was filled with the dosing solution by drawing the solution through the catheter tube. The catheter tube was wiped dry. K-Y jelly was applied to the tip, avoiding contact with the eye of the tube, and the tube was inserted into the colon through the anus until the tube was no longer visible. The solution was injected by pressing the syringe plunger, and the tube was removed.

Blood samples were collected serially from the tail artery, typically 0, 15, 30, 45, 60 and 90 minutes after oral administration and 0, 10, 20, 30, 60 and 90 minutes after IC administration. Serum PTH concentrations were quantified by a PTH radioimmunoassay kit (Kit # RIK 6101 from Peninsula Laboratories, Inc., San Carlos, CA). Previous studies indicated baseline values of about zero. Results from the five rats in each group were averaged for each time point. The maximum is reported below in Table 4.

**Table 4**

| Oral/Intracolonic Delivery of PTH in Rats | | | | | | |
|---|---|---|---|---|---|---|
| Compound | Method of Administration | Volume dose (ml/kg) | Compound Dose (mg/kg) | PTH Dose (µg/kg) | Mean Peak Serum [PTH] (pg/ml) ± SD | AUC |
| 2 | PO | 1 | 100 | 200 | 498.55 ± 237.83 | |
| 3 | PO | 1 | 100 | 200 | 57.88 ± | 2717.51 |
| 4 | PO | 1 | 100 | 200 | 330.74 ± 410.57 | 6852.12 |
| 5 | PO | 1 | 100 | 200 | 22.91 ± | 509.20 |
| 6 | PO | 1 | 100 | 200 | 14.44 ± | 716.96 |
| 7 | PO | 1 | 100 | 200 | 138.9 ± 118.46 | 4790.10 |

## Claims

1. A compound having the formula 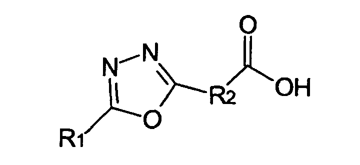 or a salt thereof, wherein
R¹ is C₃-C₁₀ cycloalkyl or naphthyl; optionally substituted with C₁-C₄ alkyl or fluoroalkyl, C₂-C₄ alkenyl, C₁-C₄ alkoxy or fluoroalkoxy, halogens, -OH, -SH, phenyl, phenoxy, -CO₂R³, -N(CH₃)₂, -NO₂, or -NH₂;
R¹ further is 2-hydroxy phenyl; optionally substituted with -OH, -Cl, -CH₃ or -OCH₃;
R² is C₁-C₂₄ alkylene, C₂-C₂₄ alkenylene, C₃-C₁₀ cycloalkylene, C₃-C₁₀ cycloalkenylene, phenylene, naphthylene, (C₁-C₁₀ alkyl)phenylene, (C₂-C₁₀ alkenyl)phenylene, (C₁-C₁₀ alkyl)naphthylene, (C₂-C₁₀ alkenyl)naphthylene, phenyl(C₁-C₁₀ alkylene), phenyl(C₂-C₁₀ alkenylene), naphthyl(C₁-C₁₀ alkylene) or naphthyl(C₂-C₁₀ alkenylene);
R² is optionally substituted with C₁-C₄ alkyl or fluoroalkyl, C₂-C₄ alkenyl, C₁-C₄ alkoxy or fluoroalkoxy, halogens, -OH, -SH, phenyl, phenoxy, -CO₂R³, -N(CH₃)₂, -NO₂, -NH₂, C₃-C₁₀ cycloalkyl, C₃-C₁₀ cycloalkenyl, aryl, (C₁-C₁₀ alk)aryl, heterocycle having 3-10 ring atoms wherein the hetero atom is one or more of N, O, S or any combination thereof, or any combination thereof;
R² being optionally interrupted by oxygen, nitrogen, sulfur, or any combination thereof; and
R³ is hydrogen, C₁-C₄ alkyl, or C₂-C₄ alkenyl.

2. The compound of claim 1, wherein
R¹ 2-hydroxy phenyl; optionally substituted with -OH, -Cl, -CH₃ or -OCH₃.

3. The compound of claim 1, wherein
R¹ C₃-C₁₀ cycloalkyl, or naphthyl; optionally substituted with halogens, -OH, -SH, C₁-C₄ alkyl or C₁-C₄ alkoxy;
R¹ is further 2-hydroxy phenyl, optionally substituted with -OH, -Cl, -CH₃ or -OCH₃;
R² is C₁-C₂₄ alkylene or (C₁-C₁₀ alkyl)phenylene.

4. The compound of claim 2, wherein
R¹ is 2-hydroxy phenyl; optionally substituted with -OH, -Cl, -CH₃ or -OCH₃
R² is C₄-C₈ alkyl.

5. A compound having the formula 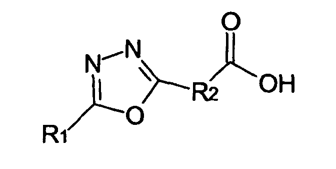 or a salt thereof, wherein
R¹ is C₂-C₁₀ alkenyl, C₂-C₁₀ alkynyl, C₃-C₁₀ cycloalkyl, phenyl, naphthyl, or aromatic heterocycle;
R¹ is optionally substituted with C₁-C₄ alkyl or fluoroalkyl, C₂-C₄ alkenyl, C₁-C₄ alkoxy or fluoroalkoxy, halogens, -OH, -SH, phenyl, phenoxy, -CO₂R³, -N(CH₃)₂, -NO₂, or -NH₂;
R² is a C₄-C₈ alkylene;
R² is optionally substituted with C₁-C₄ alkyl or fluoroalkyl, C₂-C₄ alkenyl, C₁-C₄ alkoxy or fluoroalkoxy, halogens, -OH, -SH, phenyl, phenoxy, -CO₂R³, -N(CH₃)₂, -NO₂, - NH₂, C₃-C₁₀ cycloalkyl, C₃-C₁₀ cycloalkenyl, aryl, (C₁-C₁₀ alk)aryl, heterocycle having 3-10 ring atoms wherein the hetero atom is one or more of N, O, S or any combination thereof, or any combination thereof;
R² being optionally interrupted by oxygen, nitrogen, sulfur, or any combination thereof; and
R³ is hydrogen, C₁-C₄ alkyl, or C₂-C₄ alkenyl,
with the proviso that R¹ is not 4-carboxylphenyl when R² is -(CH₂)₄-.

6. The compound of claim 5, wherein
R¹ is C₂-C₆ alkenyl, C₂-C₆ alkynyl, cycloalkyl, phenyl or naphthyl, optionally substituted with halogens, -OH, -SH, C₁-C₄ alkyl or C₁-C₄ alkoxy.

7. The compound of claim 5, wherein
R¹ is C₂-C₁₀ alkenyl, C₂-C₁₀ alkynyl, C₃-C₁₀ cycloalkyl, phenyl, napthyl, or aromatic heterocycle;
R¹ is optionally substituted with of C₁-C₄ alkyl or fluoroalkyl, C₂-C₄ alkenyl, C₁-C₄ alkoxy or fluoroalkoxy, halogens, -OH, -SH, phenyl, phenoxy, -CO₂R³, -N(CH₃)₂, or -NH₂;
R² is C₄-C₈ alkylene; and
R³ is C₁-C₄ alkyl, or C₂-C₄ alkenyl.

8. A compound having the formula 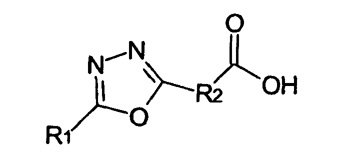 or a salt thereof, wherein
R¹ is C₂-C₁₀ alkenyl, C₂-C₁₀ alkynyl, C₃-C₁₀ cycloalkyl, phenyl, napthyl, or aromatic heterocycle;
R¹ is substituted with of C₁-C₄ alkyl or fluoroalkyl, C₂-C₄ alkenyl, C₁-C₄ fluoroalkoxy, -OH, -SH, phenyl, phenoxy, -CO₂R³, or -N(CH₃)₂:
R² is C₁-C₂₄ alkylene, C₂-C₂₄ alkenylene, C₃-C₁₀ cycloalkylene, C₃-C₁₀ cycloalkenylene, napthylene, (C₁-C₁₀ alkyl)phenylene, (C₂-C₁₀ alkenyl)phenylene, (C₁-C₁₀ alkyl)naphthylene, (C₂-C₁₀ alkenyl)naphthylene, phenyl(C₁-C₁₀ alkylene), phenyl(C₂-C₁₀ alkenylene), naphthyl(C₁-C₁₀ alkylene) or naphthyl(C₂-C₁₀alkenylene);
R² is optionally substituted with of C₁-C₄ alkyl or fluoroalkyl, C₂-C₄ alkenyl, C₁-C₄ alkoxy or fluoroalkoxy, halogens, -OH, -SH, phenyl, phenoxy, -CO₂R³, -N(CH₃)₂, -NO₂, - NH₂, C₃-C₁₀ cycloalkyl, C₃-C₁₀ cycloalkenyl, aryl, (C₁-C₁₀ alk)aryl, heterocycle having 3-10 ring atoms wherein the hetero atom is one or more of N, O, S or any combination thereof, or any combination thereof;
R² being optionally interrupted by oxygen, nitrogen, sulfur or any combination thereof; and
R³ is C₁-C₄ alkyl, or C₂-C₄ alkenyl.

9. The compound of claim 8, wherein
R¹ is C₂-C₆ alkenyl, C₂-C₆ alkynyl, C₃-C₁₀ cycloalkyl, or phenyl or naphthyl, substituted with -OH, -SH, or C₁-C₄ alkyl; and
R² is C₁-C₂₄ alkylene or (C₁-C₁₀ alkyl)phenylene.

10. A composition comprising:
(A) a biologically active agent comprising at least one protein, polypeptide, peptide, hormone, polysaccharide, mucopolysaccharide, carbohydrate, or lipid; and
(B) a compound having the formula 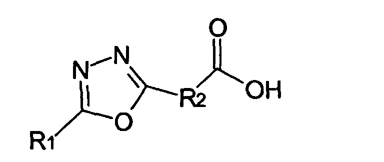
or a salt thereof, wherein
R¹ is C₁-C₁₀ alkyl, C₂-C₁₀ alkenyl, C₂-C₁₀ alkynyl, C₃-C₁₀ cycloalkyl, phenyl, naphthyl, or aromatic heterocycle;
R¹ is optionally substituted with C₁-C₄ alkyl or fluoroalkyl, C₂-C₄ alkenyl, C₁-C₄ alkoxy or fluoroalkoxy, halogens, -OH, -SH, phenyl, phenoxy, -CO₂R³, -N(CH₃)₂, -NO₂, or -NH₂;
R² is C₁ -C₂₄ alkylene, C₂-C₂₄ alkenylene, C₃-C₁₀ cycloalkylene, C₃-C₁₀ cycloalkenylene, phenylene, naphthylene, (C₁-C₁₀ alkyl)phenylene, (C₁-C₁₀ alkenyl)phenylene, (C₁-C₁₀ alkyl)naphthylene, (C₂-C₁₀ alkenyl)naphthylene, phenyl (C₁-C₁₀ alkylene), phenyl (C₂-C₁₀ alkenylene), naphthyl (C₁-C₁₀ alkylene) or naphthyl (C₂-C₁₀ alkenylene);
R² is optionally substituted with C₁-C₄ alkyl or fluoroalkyl, C₂-C₄ alkenyl, C₁-C₄ alkoxy or fluoroalkoxy, halogens, -OH, -SH, phenyl, phenoxy, -CO₂R³, -N(CH₃)₂, -NO₂, -NH₂, C₃-C₁₀ cycloalkyl, C₃-C₁₀ cycloalkenyl, aryl, (C₁-C₁₀ alk)aryl, heterocycle having 3-10 ring atoms wherein the hetero atom is one or more of N, O, S or any combination thereof, or any combination thereof;
R² being optionally interrupted by oxygen, nitrogen, sulfur, or any combination thereof; and
R³ is hydrogen, C₁-C₄ alkyl, or C₂-C₄ alkenyl.

11. The composition of claim 10, wherein
R¹ is C₁-C₆ alkyl, C₂-C₆ alkenyl, C₂-C₆ alkynyl, cycloalkyl, phenyl or naphthyl, optionally substituted with halogens, -OH, -SH, C₁-C₄ alkyl or C₁-C₄ alkoxy; and
R² is C₁-C₂₄ alkylene or (C₁-C₁₀ alkyl)phenylene.

12. The composition of claim 11, wherein
R¹ is 2-OH-phenyl, optionally substituted with -OH, -Cl, -CH₃ or -OCH₃;
R² is C₄-C₈ alkyl.

13. A composition comprising:
(A) a biologically active agent; and
(B) a compound having the formula 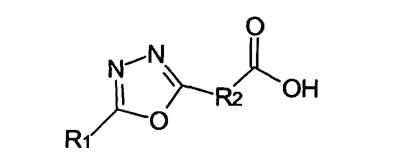
or a salt thereof, wherein
R¹ is C₃-C₁₀ cycloalkyl or naphthyl; optionally substituted with C₁-C₄ alkyl or fluoroalkyl, C₂-C₄ alkenyl, C₁-C₄ alkoxy or fluoroalkoxy, halogens, -OH, -SH, phenyl, phenoxy, -CO₂R ³, -N(CH₃)₂, -NO₂, or -NH₂;
R¹ further is 2-hydroxy phenyl; optionally substituted with -OH, -Cl, -CH₃ or -OCH₃;
R² is C₁-C₂₄ alkylene, C₂-C₂₄ alkenylene, C₃-C₁₀ cycloalkylene, C₃-C₁₀ cycloalkenylene, phenylene, naphthylene, (C₁-C₁₀ alkyl)phenylene, (C₂-C₁₀ alkenyl)phenylene, (C₁-C₁₀ alkyl)naphthylene, (C₂-C₁₀ alkenyl)naphthylene, phenyl(C₁-C₁₀ alkylene), phenyl(C₂-C₁₀ alkenylene), naphthyl(C₁-C₁₀ alkylene) or naphthyl(C₂-C₁₀ alkenylene);
R² is optionally substituted with C₁-C₄ alkyl or fluoroalkyl, C₂-C₄ alkenyl, C₁-C₄ alkoxy or fluoroalkoxy, halogens, -OH, -SH, phenyl, phenoxy, -CO₂R³, -N(CH₃)₂, -NO₂, -NH₂, C₃-C₁₀ cycloalkyl, C₃-C₁₀ cycloalkenyl, aryl, (C₁-C₁₀ alk)aryl, heterocycle having 3-10 ring atoms wherein the hetero atom is one or more of N, O, S or any combination thereof, or any combination thereof;
R² being optionally interrupted by oxygen, nitrogen, sulfur, or any combination thereof; and
R³ is hydrogen, C₁-C₄ alkyl, or C₂-C₄ alkenyl.

14. The composition of claim 13, wherein
R¹ is C₃-C₁₀ cycloalkyl, or naphthyl; optionally substituted with halogens, -OH, -SH, C₁-C₄ alkyl or C₁-C₄ alkoxy;
R¹ is further 2-hydroxy phenyl; optionally substituted with -OH, -Cl, -CH₃ or -OCH₃;
R² is C₁-C₂₄ alkylene or (C₁-C₁₀ alkyl)phenylene.

15. The composition of claim 14, wherein
R¹ is 2-hydroxy phenyl, optionally substituted with -OH, -Cl, -CH₃ or -OCH₃;
R² is C₄-C₈ alkyl.

16. A composition comprising:
(A) a biologically active agent; and
(B) a compound having the formula 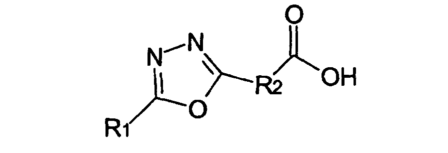
or a salt thereof, wherein
R¹ is C₁-C₁₀ alkyl, C₂-C₁₀ alkenyl, C₂-C₁₀ alkynyl, C₃-C₁₀ cycloalkyl, phenyl, naphthyl, or aromatic heterocycle;
R¹ is optionally substituted with C₁-C₄ alkyl or fluoroalkyl, C₂-C₄ alkenyl, C₁-C₄ alkoxy or fluoroalkoxy, halogens,-OH, -SH, phenyl, phenoxy, -CO₂R³, -N(CH₃)₂, -NO₂, or -NH₂;
R² is a C₄-C₈ alkylene;
R² is optionally substituted with C₁-C₄ alkyl or fluoroalkyl, C₂-C₄ alkenyl, C₁-C₄ alkoxy or fluoroalkoxy, halogens, -OH, -SH, phenyl, phenoxy, -CO₂R³, -N(CH₃)₂, -NO₂, -NH₂, C₃-C₁₀ cycloalkyl, C₃-C₁₀ cycloalkenyl, aryl, (C₁-C₁₀ alk)aryl, heterocycle having 3-10 ring atoms wherein the hetero atom is one or more of N, O, S or any combination thereof, or any combination thereof;
R² being optionally interrupted by oxygen, nitrogen, sulfur, or any combination thereof; and
R³ is hydrogen, C₁-C₄ alkyl, or C₂-C₄ alkenyl.

17. The composition of claim 16, wherein
R¹ is C₁-C₆ alkyl, C₂-C₆ alkenyl, C₂-C₆ alkynyl, cycloalkyl, phenyl or naphthyl, optionally substituted with halogens, -OH, -SH, C₁-C₄ alkyl or C₁-C₄ alkoxy.

18. The composition of any of claims 13-17, wherein the biologically active agent comprises at least one protein, polypeptide, peptide, hormone, polysaccharide, mucopolysaccharide, carbohydrate, or lipid.

19. The composition of any of claims 10-18, wherein the biologically active agent is selected from the group consisting of growth hormones, human growth hormones (hGH), recombinant human growth hormones (rhGH), bovine growth hormones, porcine growth hormones, growth hormone-releasing hormones, interferons, α-interferon, β-interferon, γ-interferon, interfeukin-1, interleukin-2, insulin, porcine insulin, bovine insulin, human insulin, human recombinant insulin, insulin-like growth factor(IGF), IGF-1, heparin, unfractionated heparin, heparinoids, dermatans, chondroitins, low molecular weight heparin, very low molecular weight heparin, ultra low molecular weight heparin, calcitonin, salmon calcitonin, eel calcitonin, human calcitonin; erythropoietin (EPO), atrial naturetic factor, antigens, monoclonal antibodies, somatostatin, protease inhibitors, adrenocorticotropin, gonadotropin releasing hormone, oxytocin, leutinizing-hormone-releasing-hormone, follicle stimulating hormone, glucocerebrosidase, thrombopoietin, filgrastim, prostaglandins, cyclosporin, vasopressin, cromolyn sodium, sodium chromoglycate, disodium chromoglycate, vancomycin, desferrioxamine (DFO), parathyroid hormone (PTH), fragments of PTH, antimicrobials, anti-fungal agents, vitamins; analogs, fragments, mimetics and polyethylene glycol (PEG)-modified derivatives of these compounds; and any combination thereof.

20. The composition of claim 19, wherein the biologically active agent comprises insulin, unfractionated heparin, low molecular weight heparin, very low molecular weight heparin, ultra low molecular weight heparin, calcitonin, PTH, EPO, hGH or combinations thereof.

21. The composition of claim 19, wherein the biologically active agent comprises salmon calcitonin.

22. A compound selected from the group consisting of 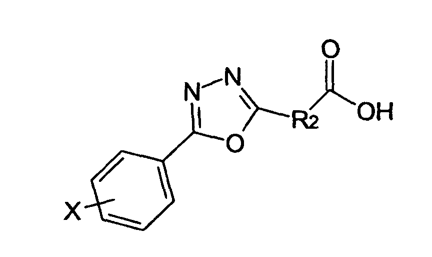
| **Cpd #** | **X** | **R**^{**2**} |
|---|---|---|
| | | |
| 1 | 2-OH | (CH₂)₈ |
| 2 | 2-OH | (CH₂)₆ |
| 3 | 2-OH | (CH₂)₄ |
| 4 | 2-OH | (CH₂)₇ |
| 5 | 2-OH-5-Cl | (CH₂)₆ |
| 6 | 2-OH-3,5-di-Cl | (CH₂)₆ |
| 7 | 2-OH-4-methyl | (CH₂)₆ |
| 8 | 2-OH-4-OMe | (CH₂)₆ |
| 9 | 2-OH-4-OMe | (CH₂)₄ |
| 10 | H | (CH₂)₆ |
| 11 | 2-Cl | (CH₂)₆ |

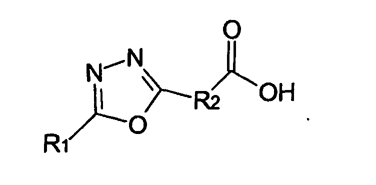
| **Cpd #** | **R**^{**1**} | **R**^{**2**} |
|---|---|---|
| | | |
| 12 | CH₃ | (CH₂)₆ |
| 13 | CH₂OH | (CH₂)₆ |
and salts thereof.

23. A composition comprising:
(A) an active agent; and
(B) at least one compound of claim 22.

24. The composition of claim 23, wherein the active agent is selected from the group consisting of a biologically active agent, a chemically active agent, and a combination thereof.

25. The composition of claim 24, wherein the biologically active agent comprises at least one protein, polypeptide, peptide, hormone, polysaccharide, mucopolysaccharide, carbohydrate, or lipid.

26. The composition of claim 24, wherein the biologically active agent is selected from the group consisting of growth hormones, human growth hormones (hGH), recombinant human growth hormones (rhGH), bovine growth hormones, porcine growth hormones, growth hormone-releasing hormones, interferons, α-interferon, β-interferon, γ-interferon, interleukin-1, interleukin-2, insulin, porcine insulin, bovine insulin, human insulin, human recombinant insulin, insulin-like growth factor (IGF), IGF-1, heparin, unfractionated heparin, heparinoids, dermatans, chondroitins, low molecular weight heparin, very low molecular weight heparin, ultra low molecular weight heparin, calcitonin, salmon-calcitonin, eel calcitonin, human calcitonin; erythropoietin (EPO), atrial naturetic factor, antigens, monoclonal antibodies, somatostatin, protease inhibitors, adrenocorticotropin, gonadotropin releasing hormone, oxytocin, leutinizing-hormone-releasing-hormone, follicle stimulating hormone, glucocerebrosidase, thrombopoietin, filgrastim, prostaglandins, cyclosporin, vasopressin, cromolyn sodium, sodium chromoglycate, disodium chromoglycate, vancomycin, desferrioxamine (DFO), parathyroid hormone (PTH), fragments of PTH, antimicrobials, anti-fungal agents, vitamins; analogs, fragments, mimetics and polyethylene glycol (PEG)-modified derivatives of these compounds; and any combination thereof.

27. The composition of claim 24, wherein the biologically active agent comprises insulin, unfractionated heparin, low molecular weight heparin, very low molecular weight heparin, ultra low molecular weight heparin, calcitonin, PTH, EPO, hGH or combinations thereof.

28. The composition of claim 24, wherein the biologically active agent comprises salmon calcitonin.

29. The composition of claim 23, wherein the compound is 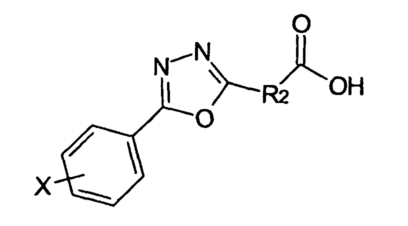 wherein
| **Cpd #** | **X** | **R**^{**2**} |
|---|---|---|
| | | |
| 3 | 2-OH | (CH₂)₄ |
or a salt thereof.

30. A dosage unit form comprising:
(A) the composition of claim 23; and
(B)
(a) an excipient
(b) a diluent,
(c) a disintegrant,
(d) a lubricant,
(e) a plasticizer,
(f) a colorant,
(g) a dosing vehicle, or
(h) any combination thereof.

31. The dosage unit form of claim 30, wherein the biologically active agent comprises at least one protein, polypeptide, peptide, hormone, polysaccharide, mucopolysaccharide, carbohydrate, or lipid.

32. The dosage unit form of claim 30, wherein the biologically active agent is selected from the group consisting of growth hormones, human growth hormones (hGH), recombinant human growth hormones (rhGH), bovine growth hormones, porcine growth hormones, growth hormone-releasing hormones, interferons, α-interferon, β-interferon, γ-interferon, interleukin-1, interleukin-2, insulin, porcine insulin, bovine insulin, human insulin, human recombinant insulin, insulin-like growth factor (IGF), IGF-1, heparin, unfractionated heparin, heparinoids, dermatans, chondroitins, low molecular weight heparin, very low molecular weight heparin, ultra low molecular weight heparin, calcitonin, salmon calcitonin, eel calcitonin, human calcitonin; erythropoietin (EPO), atrial naturetic factor, antigens, monoclonal antibodies, somatostatin, protease inhibitors, adrenocorticotropin, gonadotropin releasing hormone, oxytocin, leutinizing-hormone-releasing-hormone, follicle stimulating hormone, glucocerebrosidase, thrombopoietin, filgrastim, prostaglandins, cyclosporin, vasopressin, cromolyn sodium, sodium chromoglycate, disodium chromoglycate, vancomycin, desferrioxamine (DFO), parathyroid hormone (PTH), fragments of PTH, antimicrobials, anti-fungal agents, vitamins; analogs, fragments, mimetics and polyethylene glycol (PEG)-modified derivatives of these compounds; and any combination thereof.

33. The dosage unit form of claim 31, wherein the biologically active agent comprises insulin, unfractionated heparin, low molecular weight heparin, very low molecular weight heparin, ultra low molecular weight heparin, calcitonin, PTH, EPO, hGH, or combinations thereof.

34. The dosage unit form of claim 30, wherein the active agent comprises salmon calcitonin.

35. The dosage unit form of claim 30, wherein the dosage unit form is in the form of a tablet, a capsule, a powder or a liquid.

36. Use of the composition of claim 24, for the preparation of a medicament for the treatment of diseases.

37. A method for preparing a composition comprising mixing:
a) at least one active agent;
b) the compound of claim 22;
c) optionally, a dosing vehicle.

## Patentansprüche

1. Verbindung der Formel 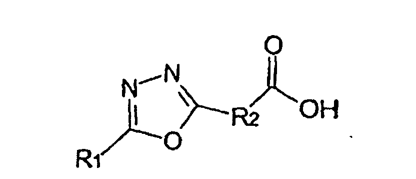 oder ein Salz davon, wobei
R¹ C₃-C₁₀-Cycloalkyl oder Naphthyl ist; gegebenenfalls substituiert mit C₁-C₄-Alkyl oder Fluoralkyl, C₂-C₄-Alkenyl, C₁-C₄-Alkoxy oder Fluoralkoxy, Halogenen, -OH, -SH, Phenyl, Phenoxy, -CO₂R ³, -N(CH₃)₂, -NO₂ oder -NH₂;
R¹ ferner 2-Hydroxyphenyl ist; gegebenenfalls substituiert mit -OH, -Cl, -CH₃ oder -OCH₃;
R² C₁-C₂₄-Alkylen, C₂-C₂₄-Alkenylen, C₃-C₁₀-Cycloalkylen, C₃-C₁₀-Cycloalkenylen, Phenylen, Naphthylen, (C₁-C₁₀-Alkyl)phenylen, (C₂-C₁₀-Alkenyl)phenylen, (C₁-C₁₀-Alkyl)naphthylen, (C₂-C₁₀-Alkenyl)naphthylen, Phenyl(C₁-C₁₀-alkylen), Phenyl(C₂-C₁₀-alkenylen), Naphthyl(C₁-C₁₀-alkylen) oder Naphthyl(C₂-C₁₀-alkenylen) ist;
R² gegebenenfalls mit C₁-C₄-Alkyl oder Fluoralkyl, C₂-C₄-Alkenyl, C₁-C₄-Alkoxy oder Fluoralkoxy, Halogenen, -OH, -SH, Phenyl, Phenoxy, -CO₂R³, -N(CH₃)₂, -NO₂, -NH₂, C₃-C₁₀-Cycloalkyl, C₃-C₁₀-Cycloalkenyl, Aryl, (C₁-C₁₀-Alk)aryl, Heterocyclus mit 3 - 10 Ringatomen, wobei das Heteroatom eins oder mehr aus N, O, S oder eine beliebige Kombination davon ist, oder einer beliebigen Kombination davon substituiert ist.
R² gegebenenfalls durch Sauerstoff, Stickstoff, Schwefel oder eine beliebige Kombination davon unterbrochen ist; und
R³ Wasserstoff, C₁-C₄-Alkyl oder C₂-C₄-Alkenyl ist.

2. Verbindung nach Anspruch 1, wobei
R¹ 2-Hydroxyphenyl ist; gegebenenfalls substituiert mit -OH, -Cl, -CH₃ oder -OCH₃.

3. Verbindung nach Anspruch 1, wobei
R¹ C₃-C₁₀-Cycloalkyl oder Naphthyl ist; gegebenenfalls substituiert mit Halogenen, -OH, -SH, C₁-C₄-Alkyl oder C₁-C₄-Alkoxy;
R¹ ferner 2-Hydroxyphenyl ist, gegebenenfalls substituiert mit -OH, -Cl, -CH₃ oder -OCH₃;
R² C₁-C₂₄-Alkylen oder (C₁-C₁₀-Alkyl)phenylen ist.

4. Verbindung nach Anspruch 2, wobei
R¹ 2-Hydroxyphenyl ist; gegebenenfalls substituiert mit -OH, -Cl, -CH₃ oder -OCH₃;
R² C₄-C₈-Alkyl ist.

5. Verbindung der Formel 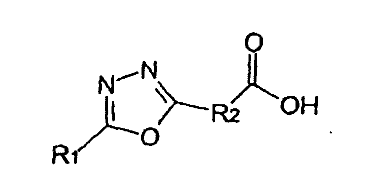 oder ein Salz davon, wobei
R¹ C₂-C₁₀-Alkenyl, C₂-C₁₀-Alkinyl, C₃-C₁₀-Cycloalkyl, Phenyl, Naphthyl oder ein aromatischer Heterocyclus ist;
R¹ gegebenenfalls mit C₁-C₄-Alkyl oder Fluoralkyl, C₂-C₄-Alkenyl, C₁-C₄-Alkoxy oder Fluoralkoxy, Halogenen, -OH, -SH, Phenyl, Phenoxy, -CO₂R³, -N(CH₃)₂, -NO₂ oder -NH₂ substituiert ist;
R² ein C₄-C₈-Alkylen ist;
R² gegebenenfalls mit C₁-C₄-Alkyl oder Fluoralkyl, C₂-C₄-Alkenyl, C₁-C₄-Alkoxy oder Fluoralkoxy, Halogenen, -OH, -SH, Phenyl, Phenoxy, -CO₂R ³, -N(CH₃)₂, -NO₂, -NH₂, C₃-C₁₀-Cycloalkyl, C₃-C₁₀-Cycloalkenyl, Aryl, (C₁-C₁₀-Alk)aryl, Heterocyclus mit 3 - 10 Ringatomen, wobei das Heteroatom eins oder mehr aus N, O, S oder eine beliebige Kombination davon ist, oder einer beliebigen Kombination davon substituiert ist.
R² gegebenenfalls durch Sauerstoff, Stickstoff, Schwefel oder eine beliebige Kombination davon unterbrochen ist; und
R³ Wasserstoff, C₁-C₄-Alkyl oder C₂-C₄-Alkenyl ist, mit der Maßgabe, dass R¹ nicht 4-Carboxyphenyl ist, wenn R² -(CH₂)₄- ist.

6. Verbindung nach Anspruch 5, wobei
R¹ C₂-C₆-Alkenyl, C₂-C₆-Alkinyl, Cycloalkyl, Phenyl oder Naphthyl ist, gegebenenfalls substituiert mit Halogenen, -OH, -SH, C₁-C₄-Alkyl oder C₁-C₄-Alkoxy.

7. Verbindung nach Anspruch 5, wobei
R¹ C₂-C₁₀-Alkenyl, C₂-C₁₀-Alkinyl, C₃-C₁₀-Cycloalkyl, Phenyl, Naphthyl oder ein aromatischer Heterocyclus ist;
R¹ gegebenenfalls mit C₁-C₄-Alkyl oder Fluoralkyl, C₂-C₄-Alkenyl, C₁-C₄-Alkoxy oder Fluoralkoxy, Halogenen, -OH, -SH, Phenyl, Phenoxy, -CO₂R ³, -N(CH₃)₂ oder -NH₂ substituiert ist;
R² C₄-C₈-Alkylen ist; und
R³ C₁-C₄-Alkyl oder C₂-C₄-Alkenyl ist.

8. Verbindung der Formel 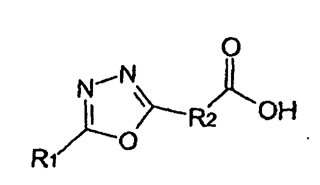 oder ein Salz davon, wobei
R¹ C₂-C₁₀-Alkenyl, C₂-C₁₀-Alkinyl, C₃-C₁₀-Cycloalkyl, Phenyl, Naphthyl oder ein aromatischer Heterocyclus ist;
R¹ mit C₁-C₄-Alkyl oder Fluoralkyl, C₂-C₄-Alkenyl, C₁-C₄-Fluoralkoxy, -OH, -SH, Phenyl, Phenoxy, -CO₂R³ oder -N(CH₃)₂ substituiert ist;
R² C₁-C₂₄-Alkylen, C₂-C₂₄-Alkenylen, C₃-C₁₀-Cycloalkylen, C₃-C₁₀-Cycloalkenylen, Naphthylen, (C₁-C₁₀-Alkyl)phenylen, (C₂-C₁₀-Alkenyl)phenylen, (C₁-C₁₀-Alkyl)naphthylen, (C₂-C₁₀-Alkenyl)naphthylen, Phenyl(C₁-C₁₀-alkylen), Phenyl(C₂-C₁₀-alkenylen), Naphthyl-(C₁-C₁₀-alkylen) oder Naphthyl(C₂-C₁₀-alkenylen) ist;
R² gegebenenfalls mit C₁-C₄-Alkyl oder Fluoralkyl, C₂-C₄-Alkenyl, C₁-C₄-Alkoxy oder Fluoralkoxy, Halogenen, -OH, -SH, Phenyl, Phenoxy, -CO₂R³, -N(CH₃)₂, -NO₂, -NH₂, C₃-C₁₀-Cycloalkyl, C₃-C₁₀-Cycloalkenyl, Aryl, (C₁-C₁₀-Alk)aryl, Heterocyclus mit 3 - 10 Ringatomen, wobei das Heteroatom eins oder mehr aus N, O, S oder eine beliebige Kombination davon ist, oder einer beliebigen Kombination davon substituiert ist.
R² gegebenenfalls durch Sauerstoff, Stickstoff, Schwefel oder eine beliebige Kombination davon unterbrochen ist; und
R³ C₁-C₄-Alkyl oder C₂-C₄-Alkenyl ist.

9. Verbindung nach Anspruch 8, wobei
R¹ C₂-C₆-Alkenyl, C₂-C₆-Alkinyl, C₃-C₁₀-Cycloalkyl oder Phenyl oder Naphthyl ist, substituiert mit -OH, -SH oder C₁-C₄-Alkyl; und
R² C₁-C₂₄-Alkylen oder (C₁-C₁₀-Alkyl)phenylen ist.

10. Zusammensetzung umfassend:
(A) einen biologischen Wirkstoff, umfassend mindestens ein Protein, Polypeptid, Peptid, Hormon, Polysaccharid, Mucopolysaccharid, Kohlenhydrat oder Lipid; und
(B) eine Verbindung der Formel 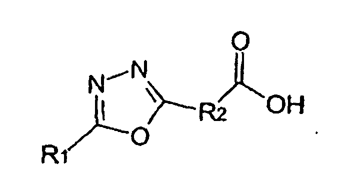
oder ein Salz davon, wobei
R¹ C₁-C₁₀-Alkyl, C₂-C₁₀-Alkenyl, C₂-C₁₀-Alkinyl, C₃-C₁₀-Cycloalkyl, Phenyl, Naphthyl oder ein aromatischer Heterocyclus ist;
R¹ gegebenenfalls mit C₁-C₄-Alkyl oder Fluoralkyl, C₂-C₄-Alkenyl, C₁-C₄-Alkoxy oder Fluoralkoxy, Halogenen, -OH, -SH, Phenyl, Phenoxy, -CO₂R³, -N(CH₃)₂, -NO₂ oder -NH₂ substituiert ist;
R² C₁-C₂₄-Alkylen, C₂-C₂₄-Alkenylen, C₃-C₁₀-Cycloalkylen, C₃-C₁₀-Cycloalkenylen, Phenylen, Naphthylen, (C₁-C₁₀-Alkyl)phenylen, (C₁-C₁₀-Alkenyl)phenylen, (C₁-C₁₀-Alkyl)naphthylen, (C₂-C₁₀-Alkenyl)naphthylen, Phenyl(C₁-C₁₀-alkylen), Phenyl(C₂-C₁₀-alkenylen), Naphthyl(C₁-C₁₀-alkylen) oder Naphthyl(C₂-C₁₀-alkenylen) ist;
R² gegebenenfalls mit C₁-C₄-Alkyl oder Fluoralkyl, C₂-C₄-Alkenyl, C₁-C₄-Alkoxy oder Fluoralkoxy, Halogenen, -OH, -SH, Phenyl, Phenoxy, -CO₂R³, -N(CH₃)₂, -NO₂, -NH₂, C₃-C₁₀-Cycloalkyl, C₃-C₁₀-Cycloalkenyl, Aryl, (C₁-C₁₀-Alk)aryl, Heterocyclus mit 3 - 10 Ringatomen, wobei das Heteroatom eins oder mehr aus N, O, S oder eine Kombination davon ist, oder einer beliebigen Kombination davon substituiert ist.
R² gegebenenfalls durch Sauerstoff, Stickstoff, Schwefel oder eine beliebige Kombination davon unterbrochen ist; und
R³ Wasserstoff, C₁-C₄-Alkyl oder C₂-C₄-Alkenyl ist.

11. Zusammensetzung nach Anspruch 10, wobei
R¹ C₁-C₆-Alkyl, C₂-C₆-Alkenyl, C₂-C₆-Alkinyl, Cycloalkyl, Phenyl oder Naphthyl ist, gegebenenfalls substituiert mit Halogenen, -OH, -SH, C₁-C₄-Alkyl oder C₁-C₄-Alkoxy; und
R² C₁-C₂₄-Alkylen oder (C₁-C₁₀-Alkyl)phenylen ist.

12. Zusammensetzung nach Anspruch 11, wobei
R¹ 2-OH-Phenyl ist, gegebenenfalls substituiert mit -OH, -Cl, -CH₃ oder -OCH₃;
R² C₄-C₈-Alkyl ist.

13. Zusammensetzung umfassend:
(A) einen biologischen Wirkstoff; und
(B) eine Verbindung der Formel 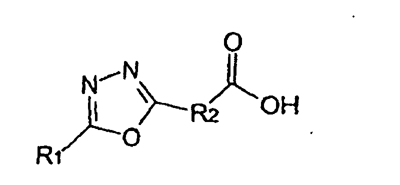
oder ein Salz davon, wobei
R¹ C₃-C₁₀-Cycloalkyl oder Naphthyl ist; gegebenenfalls substituiert mit C₁-C₄-Alkyl oder Fluoralkyl, C₂-C₄-Alkenyl, C₁-C₄-Alkoxy oder Fluoralkoxy, Halogenen, -OH, -SH, Phenyl, Phenoxy, -CO₂R³, -N(CH₃)₂, -NO₂ oder -NH₂;
R¹ ferner 2-Hydroxyphenyl ist; gegebenenfalls substituiert mit -OH, -Cl, -CH₃ oder -OCH₃;
R² C₁-C₂₄-Alkylen, C₂-C₂₄-Alkenylen, C₃-C₁₀-Cycloalkylen, C₃-C₁₀-Cycloalkenylen, Phenylen, Naphthylen, (C₁-C₁₀-Alkyl)phenylen, (C₂-C₁₀-Alkenyl)phenylen, (C₁-C₁₀-Alkyl)naphthylen, (C₂-C₁₀-Alkenyl)naphthylen, Phenyl(C₁-C₁₀-alkylen), Phenyl(C₂-C₁₀-alkenylen), Naphthyl(C₁-C₁₀-alkylen) oder Naphthyl(C₂-C₁₀-alkenylen) ist;
R² gegebenenfalls mit C₁-C₄-Alkyl oder Fluoralkyl, C₂-C₄-Alkenyl, C₁-C₄-Alkoxy oder Fluoralkoxy, Halogenen, -OH, -SH, Phenyl, Phenoxy, -CO₂R³, -N(CH₃)₂, -NO₂, -NH₂, C₃-C₁₀-Cycloalkyl, C₃-C₁₀-Cycloalkenyl, Aryl, (C₁-C₁₀-Alk)aryl, Heterocyclus mit 3 - 10 Ringatomen, wobei das Heteroatom eins oder mehr aus N, O, S oder eine beliebige Kombination davon ist, oder einer beliebigen Kombination davon substituiert ist;
R² gegebenenfalls durch Sauerstoff, Stickstoff, Schwefel oder eine beliebige Kombination davon unterbrochen ist; und
R³ Wasserstoff, C₁-C₄-Alkyl oder C₂-C₄-Alkenyl ist.

14. Zusammensetzung nach Anspruch 13, wobei
R¹ C₃-C₁₀-Cycloalkyl oder Naphthyl ist; gegebenenfalls substituiert mit Halogenen, -OH, -SH, C₁-C₄-Alkyl oder C₁-C₄-Alkoxy;
R¹ ferner 2-Hydroxyphenyl ist; gegebenenfalls substituiert mit -OH, -Cl, -CH₃ oder -OCH₃;
R² C₁-C₂₄-Alkylen oder (C₁-C₁₀-Alkyl)phenylen ist.

15. Zusammensetzung nach Anspruch 14, wobei
R¹ 2-Hydroxyphenyl ist, gegebenenfalls substituiert mit -OH, -Cl, -CH₃ oder -OCH₃;
R² C₄-C₈-Alkyl ist.

16. Zusammensetzung umfassend:
(A) einen biologischen Wirkstoff; und
(B) eine Verbindung der Formel 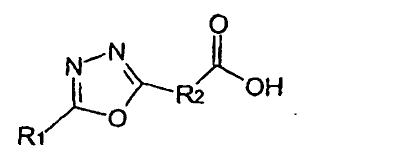
oder ein Salz davon, wobei
R¹ C₁-C₁₀-Alkyl, C₂-C₁₀-Alkenyl, C₂-C₁₀-Alkinyl, C₃-C₁₀-Cycloalkyl, Phenyl, Naphthyl oder ein aromatischer Heterocyclus ist;
R¹ gegebenenfalls mit C₁-C₄-Alkyl oder Fluoralkyl, C₂-C₄-Alkenyl, C₁-C₄-Alkoxy oder Fluoralkoxy, Halogenen, -OH, -SH, Phenyl, Phenoxy, -CO₂R³, -N(CH₃)₂, -NO₂ oder -NH₂ substituiert ist;
R² ein C₄-C₈-Alkylen ist;
R² gegebenenfalls mit C₁-C₄-Alkyl oder Fluoralkyl, C₂-C₄-Alkenyl, C₁-C₄-Alkoxy oder Fluoralkoxy, Halogenen, -OH, -SH, Phenyl, Phenoxy, -CO₂R³, -N(CH₃)₂, -NO₂, -NH₂, C₃-C₁₀-Cycloalkyl, C₃-C₁₀-Cycloalkenyl, Aryl, (C₁-C₁₀-Alk)aryl, Heterocyclus mit 3 - 10 Ringatomen, wobei das Heteroatom eins oder mehr aus N, O, S oder eine beliebige Kombination davon ist, oder einer beliebigen Kombination davon substituiert ist;
R² gegebenenfalls durch Sauerstoff, Stickstoff, Schwefel oder eine beliebige Kombination davon unterbrochen ist; und
R³ Wasserstoff, C₁-C₄-Alkyl oder C₂-C₄-Alkenyl ist.

17. Zusammensetzung nach Anspruch 16, wobei
R¹ C₁-C₆-Alkyl, C₂-C₆-Alkenyl, C₂-C₆-Alkinyl, Cycloalkyl, Phenyl oder Naphthyl ist, gegebenenfalls substituiert mit Halogenen, -OH, -SH, C₁-C₄-Alkyl oder C₁-C₄-Alkoxy.

18. Zusammensetzung nach einem der Ansprüche 13 - 17, wobei der biologische Wirkstoff mindestens ein Protein, Polypeptid, Peptid, Hormon, Polysaccharid, Mucopolysaccharid, Kohlenhydrat oder Lipid umfasst.

19. Zusammensetzung nach einem der Ansprüche 10 - 18, wobei der biologische Wirkstoff aus der Gruppe bestehend aus Wachstumshormonen, humanen Wachstumshormonen (hGH), rekombinanten humanen Wachstumshormonen (rhGH), Rinderwachstumshormonen, Schweinewachstumshormonen, Wachstumshormon-freisetzenden Hormonen, Interferonen, α-Interferon, β-Interferon, γ-Interferon, Interleukin-1, Interleukin-2, Insulin, Schweineinsulin, Rinderinsulin, Humaninsulin, rekombinantem Humaninsulin, insulinähnlichern Wachstumsfaktor (IGF), IGF-1, Heparin, unfraktioniertem Heparin, Heparinoiden, Dermatanen, Chondroitinen, niedermolekularem Heparin, sehr niedermolekularem Heparin, ultraniedermolekularem Heparin, Calcitonin, Lachs-Calcitonin, Aal-Calcitonin, Human-Calcitonin; Erythropoietin (EPO), atrialem natriuretischem Faktor, Antigenen, monoklonalen Antikörpern, Somatostatin, Proteaseinhibitoren, Adrenocorticotropin, Gonadotropin-freisetzendem Hormon, Oxytocin, Luteinisierungshormon-freisetzendem Hormon, follikelstimulierendem Hormon, Glucocerebrosidase, Thrombopoietin, Filgrastim, Prostaglandinen, Cyclosporin, Vasopressin, Cromolynnatrium, Natriumchromoglycat, Dinatriumchromoglycat, Vancomycin, Desferrioxamin (DFO), Parathormon (PTH), Fragmenten des Parathormons, antimikrobiellen Mitteln, Antimykotika, Vitaminen; Analoga, Fragmenten, Mimetika und Polyethylenglycol (PEG)-modifizierten Derivaten dieser Verbindungen; und einer beliebigen Kombination davon ausgewählt ist.

20. Zusammensetzung nach Anspruch 19, wobei der biologische Wirkstoff Insulin, unfraktioniertes Heparin, niedermolekulares Heparin, sehr niedermolekulares Heparin, ultraniedermolekulares Heparin, Calcitonin, PTH, EPO, hGH oder Kombinationen davon umfasst.

21. Zusammensetzung nach Anspruch 19, wobei der biologische Wirkstoff Lachs-Calcitonin umfasst.

22. Verbindung ausgewählt aus der Gruppe bestehend aus 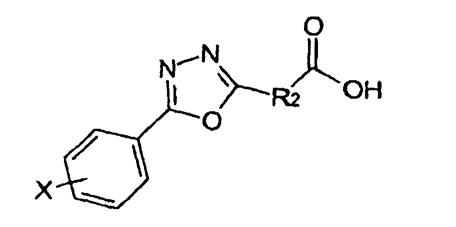
| **Verb. #** | **X** | **R**^{**2**} |
|---|---|---|
| | | |
| 1 | 2-OH | (CH₂)₈ |
| 2 | 2-OH | (CH₂)₆ |
| 3 | 2-OH | (CH₂)₄ |
| 4 | 2-OH | (CH₂)₇ |
| 5 | 2-OH-5-Cl | (CH₂)₆ |
| 6 | 2-OH-3,5-di-Cl | (CH₂)₆ |
| 7 | 2-OH-4-Methyl | (CH₂)₆ |
| 8 | 2-O H-4-OMe | (CH₂)₆ |
| 9 | 2-OH-4-OMe | (CH₂)₄ |
| 10 | H | (CH₂)₆ |
| 11 | 2-Cl | (CH₂)₆ |

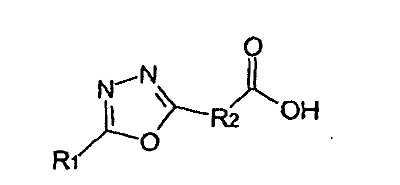
| **Verb. #** | **R**^{**1**} | **R**^{**2**} |
|---|---|---|
| | | |
| 12 | CH₃ | (CH₂)₆ |
| 13 | CH₂OH | (CH₂)₆ |
und Salzen davon.

23. Zusammensetzung umfassend:
(A) einen Wirkstoff; und
(B) mindestens eine Verbindung nach Anspruch 22.

24. Zusammensetzung nach Anspruch 23, wobei der Wirkstoff aus der Gruppe bestehend aus einem biologischen Wirkstoff, einem chemischen Wirkstoff und einer Kombination davon ausgewählt ist.

25. Zusammensetzung nach Anspruch 24, wobei der biologische Wirkstoff mindestens ein Protein, Polypeptid, Peptid, Hormon, Polysaccharid, Mucopolysaccharid, Kohlenhydrat oder Lipid umfasst.

26. Zusammensetzung nach Anspruch 24, wobei der biologische Wirkstoff aus der Gruppe bestehend aus Wachstumshormonen, humanen Wachstumshormonen (hGH), rekombinanten humanen Wachstumshormonen (rhGH), Rinderwachstumshormonen, Schweinewachstumshormonen, Wachstumshormon-freisetzenden Hormonen, Interferonen, α-Interferon, β-Interferon, γ-Interferon, Interleukin-1, Interleukin-2, Insulin, Schweineinsulin, Rinderinsulin, Humaninsulin, rekombinantem Humaninsulin, insulinähnlichem Wachstumsfaktor (IGF), IGF-1, Heparin, unfraktioniertem Heparin, Heparinoiden, Dermatanen, Chondroitinen, niedermolekularem Heparin, sehr niedermolekularem Heparin, ultraniedermolekularem Heparin, Calcitonin, Lachs-Calcitonin, Aal-Calcitonin, Human-Calcitonin; Erythropoietin (EPO), atrialem natriuretischem Faktor, Antigenen, monoklonalen Antikörpern, Somatostatin, Proteaseinhibitoren, Adrenocorticotropin, Gonadotropin-freisetzendem Hormon, Oxytocin, Luteinisierungshormon-freisetzendem Hormon, follikelstimulierendem Hormon, Glucocerebrosidase, Thrombopoietin, Filgrastim, Prostaglandinen, Cyclosporin, Vasopressin, Cromolynnatrium, Natriumchromoglycat, Dinatriumchromoglycat, Vancomycin, Desferrioxamin (DFO), Parathormon (PTH), Fragmenten des Parathormons, antimikrobiellen Mitteln, Antimykotika, Vitaminen; Analoga, Fragmenten, Mimetika und Polyethylenglycol (PEG)-modifizierten Derivaten dieser Verbindungen; und einer beliebigen Kombination davon ausgewählt ist.

27. Zusammensetzung nach Anspruch 24, wobei der biologische Wirkstoff Insulin, unfraktioniertes Heparin, niedermolekulares Heparin, sehr niedermolekulares Heparin, ultraniedermolekulares Heparin, Calcitonin, PTH, EPO, hGH oder Kombinationen davon umfasst.

28. Zusammensetzung nach Anspruch 24, wobei der biologische Wirkstoff Lachs-Calcitonin umfasst.

29. Zusammensetzung nach Anspruch 23, wobei die Verbindung 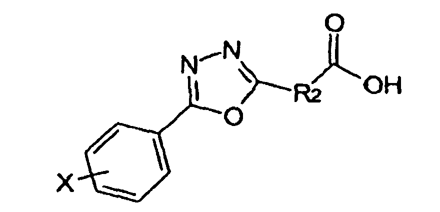 wobei
| **Verb.#** | **X** | **R**^{**2**} |
|---|---|---|
| | | |
| 3 | 2-OH | (CH₂)₄ |
oder ein Salz davon ist.

30. Dosierungseinheitsform umfassend:
(A) die Zusammensetzung nach Anspruch 23; und
(B)
(a) einen Exzipienten
(b) ein Verdünnungsmittel
(c) ein Sprengmittel
(d) ein Gleitmittel
(e) einen Weichmacher
(f) einen Farbstoff
(g) ein Dosierhilfsmittel oder
(h) eine beliebige Kombination davon.

31. Dosierungseinheitsform nach Anspruch 30, wobei der biologische Wirkstoff mindestens ein Protein, Polypeptid, Peptid, Hormon, Polysaccharid, Mucopolysaccharid, Kohlenhydrat oder Lipid umfasst.

32. Dosierungseinheitsform nach Anspruch 30, wobei der biologische Wirkstoff aus der Gruppe bestehend aus Wachstumshormonen, humanen Wachstumshormonen (hGH), rekombinanten humanen Wachstumshormonen (rhGH), Rinderwachstumshormonen, Schweinewachstumshormonen, Wachstumshormon-freisetzenden Hormonen, Interferonen, α-Interferon, β-Interferon, γ-Interferon, Interleukin-1, Interleukin-2, Insulin, Schweineinsulin, Rinderinsulin, Humaninsulin, rekombinantem Humaninsulin, insulinähnlichem Wachstumsfaktor (IGF), IGF-1, Heparin, unfraktioniertem Heparin, Heparinoiden, Dermatanen, Chondroitinen, niedermolekularem Heparin, sehr niedermolekularem Heparin, ultraniedermolekularem Heparin, Calcitonin, Lachs-Calcitonin, Aal-Calcitonin, Human-Calcitonin; Erythropoietin (EPO), atrialem natriuretischem Faktor, Antigenen, monoklonalen Antikörpern, Somatostatin, Proteaseinhibitoren, Adrenocorticotropin, Gonadotropin-freisetzendem Hormon, Oxytocin, Luteinisierungshormon-freisetzendem Hormon, follikelstimulierendem Hormon, Glucocerebrosidase, Thrombopoietin, Filgrastim, Prostaglandinen, Cyclosporin, Vasopressin, Cromolynnatrium, Natriumchromoglycat, Dinatriumchromoglycat, Vancomycin, Desferrioxamin (DFO), Parathormon (PTH), Fragmenten des Parathormons, antimikrobiellen Mitteln, Antimykotika, Vitaminen; Analoga, Fragmenten, Mimetika und Polyethylenglycol (PEG)-modifizierten Derivaten dieser Verbindungen; und einer beliebigen Kombination davon ausgewählt ist.

33. Dosierungseinheitsform nach Anspruch 31, wobei der biologische Wirkstoff Insulin, unfraktioniertes Heparin, niedermolekulares Heparin, sehr niedermolekulares Heparin, ultraniedermolekulares Heparin, Calcitonin, PTH, EPO, hGH oder Kombinationen davon umfasst.

34. Dosierungseinheitsform nach Anspruch 30, wobei der Wirkstoff Lachs-Calcitonin umfasst.

35. Dosierungseinheitsform nach Anspruch 30, wobei die Dosierungseinheitsform in Form einer Tablette, einer Kapsel, eines Pulvers oder einer Flüssigkeit ist.

36. Verwendung der Zusammensetzung nach Anspruch 24 zur Herstellung eines Medikaments zur Behandlung von Erkrankungen.

37. Verfahren zum Herstellen einer Zusammensetzung, umfassend das Mischen:
a) mindestens eines Wirkstoffs;
b) der Verbindung nach Anspruch 22;
c) gegebenenfalls eines Dosierhilfsmittels.

## Revendications

1. Composé ayant la formule 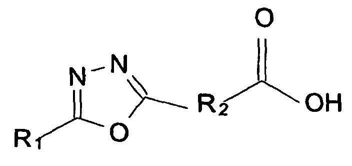 ou un sel de celui-ci, dans lequel
R¹ est un groupe cycloalkyle en C₃ à C₁₀ ou naphtyle; éventuellement substitué par un groupe alkyle ou fluoroalkyle en C₁ à C₄, alcényle en C₂ à C₄, alcoxy ou fluoroalcoxy en C₁ à C₄, des groupes halogènes, -OH, -SH, phényle, phénoxy, -CO₂R³, -N(CH₃)₂, -NO₂ ou - NH₂;
R¹ est en outre un groupe 2-hydroxyphényle; éventuellement substitué par -OH, -Cl, -CH₃, ou -OCH₃;
R² est un groupe alkylène en C₁ à C₂₄, alcénylène en C₂ à C₂₄, cycloalkylène en C₃ à C₁₀, cycloalcénylène en C₃ à C₁₀, phénylène, naphtylène, (alkyl en C₁ à C₁₀)phénylène, (alcényl en C₂ à C₁₀)phénylène, (alkyl en C₁ à C₁₀)naphtylène, (alcényl en C₂ à C₁₀)naphtylène, phényl(alkylène en C₁ à C₁₀), phényl(alcénylène en C₂ à C₁₀), naphtyl(alkylène en C₁ à C₁₀) ou naphtyl(alcénylène en C₂ à C₁₀);
R² est éventuellement substitué par un groupe alkyle ou fluoroalkyle en C₁ à C₄, alcényle en C₂ à C₄, alcoxy ou fluoroalcoxy en C₁ à C₄, des groupes halogènes, -OH, -SH, phényle, phénoxy, -CO₂R³, - N(CH₃)₂, -NO₂ ou -NH₂; cycloalkyle en C₃ à C₁₀, cycloalcényle en C₃ à C₁₀, aryle, (alkyl en C₁ à C₁₀)aryle, un hétérocycle ayant de 3 à 10 chaînons où l'hétéroatome est un ou plusieurs atomes correspondant à N, O, S ou toute combinaison de ceux-ci, ou par toute combinaison de tels substituants,
R² étant éventuellement entrecoupé par un atome d'oxygène, d'azote, de soufre, ou toute ou toute combinaison de ceux-ci, et
R³ est un atome d'hydrogène, un groupe alkyle en C₁ à C₄, ou alcényle en C₂ à C₄.

2. Composé selon la revendication 1, dans lequel
R¹ est un groupe 2-hydroxyphényle; éventuellement substitué par -OH, -Cl, -CH₃, ou -OCH₃.

3. Composé selon la revendication 1, dans lequel
R¹ est un groupe cycloalkyle en C₃ à C₁₀ ou naphtyle; éventuellement substitué par des groupes halogènes, -OH, -SH, alkyle en C₁ à C₄ ou alcoxy en C₁ à C₄;
R¹ est en outre un groupe 2-hydroxyphényle, éventuellement substitué par -OH, -Cl, -CH₃, ou -OCH₃;
R² est un groupe alkylène en C₁ à C₂₄ ou (alkyl en C₁ à C₁₀)phénylène.

4. Composé selon la revendication 2, dans lequel
R¹ est un groupe 2-hydroxyphényle; éventuellement substitué par -OH, -CI, -CH₃, ou -OCH₃,
R² est un groupe alkyle en C₄ à C₈.

5. Composé ayant la formule 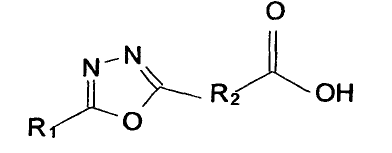 ou un sel de celui-ci, dans lequel
R¹ est un groupe alcényle en C₂ à C₁₀, alcynyle en C₂ à C₁₀, cycloalkyle en C₃ à C₁₀, phényle, naphtyle, ou un hétérocycle aromatique;
R¹ est éventuellement substitué par un groupe alkyle ou fluoroalkyle en C₁ à C₄, alcényle en C₂ à C₄, alcoxy ou fluoroalcoxy en C₁ à C₄, des groupes halogènes, -OH, -SH, phényle, phénoxy, -CO₂R³, - N(CH₃)₂, -NO₂ ou -NH₂;
R² est un groupe alkylène en C₄ à C₈;
R² est éventuellement substitué par un groupe alkyle ou fluoroalkyle en C₁ à C₄, alcényle en C₂ à C₄, alcoxy ou fluoroalcoxy en C₁ à C₄, des groupes halogènes, -OH, -SH, phényle, phénoxy, -CO₂R³, -N(CH₃)₂, -NO₂ ou -NH₂; cycloalkyle en C₃ à C₁₀, cycloalcényle en C₃ à C₁₀, aryle, (alkyl en C₁ à C₁₀)aryle, un hétérocycle ayant de 3 à 10 chaînons où l'hétéroatome est un ou plusieurs atomes correspondant à N, O, S ou toute combinaison de ceux-ci, ou par toute combinaison de tels substituants,
R² étant éventuellement entrecoupé par un atome d'oxygène, d'azote, de soufre, ou toute combinaison de ceux-ci, et
R³ est un atome d'hydrogène, un groupe alkyle en C₁ à C₄, ou alcényle en C₂ à C₄,
à la condition que R¹ ne soit pas un groupe 4-carboxyphényle lorsque R² est -(CH₂)₄-.

6. Composé selon la revendication 5, dans lequel
R¹ est un groupe alcényle en C₂ à C₆, alcynyle en C₂ à C₆, cycloalkyle, phényle ou naphtyle, éventuellement substitué par des groupes halogènes, -OH, -SH, alkyle en C₁ à C₄ ou alcoxy en C₁ à C₄.

7. Composé selon la revendication 5, dans lequel
R¹ est un groupe alcényle en C₂ à C₁₀, alcynyle en C₂ à C₁₀, cycloalkyle en C₃ à C₁₀, phényle, naphtyle, ou un hétérocycle aromatique;
R¹ est éventuellement substitué par un groupe alkyle ou fluoroalkyle en C₁ à C₄, alcényle en C₂ à C₄, alcoxy ou fluoroalcoxy en C₁ à C₄, des groupes halogènes, -OH, -SH, phényle, phénoxy, -CO₂R³, -N(CH₃)₂ ou -NH₂;
R² est un groupe alkylène en C₄ à C₈;
R³ est un groupe alkyle en C₁ à C₄, ou alcényle en C₂ à C₄.

8. Composé ayant la formule 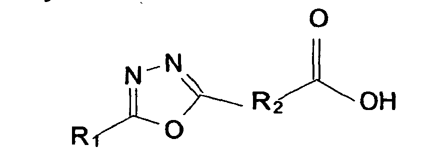 ou un sel de celui-ci, dans lequel
R¹ est un groupe alcényle en C₂ à C₁₀, alcynyle en C₂ à C₁₀, cycloalkyle en C₃ à C₁₀, phényle, naphtyle, ou un hétérocycle aromatique;
R¹ est substitué par un groupe alkyle ou fluoroalkyle en C₁ à C₄, alcényle en C₂ à C₄, fluoroalcoxy en C₁ à C₄, -OH, -SH, phényle, phénoxy, -CO₂ R³, -N(CH₃)₂,
R² est un groupe alkylène en C₁ à C₂₄, alcénylène en C₂ à C₂₄, cycloalkylène en C₃ à C₁₀, cycloalcénylène en C₃ à C₁₀, naphtylène, (alkyl en C₁ à C₁₀)phénylène, (alcényl en C₂ à C₁₀)phénylène, (alkyl en C₁ à C₁₀)naphtylène, (alcényl en C₂ à C₁₀)naphtylène, phényl (alényl en C₁ à C₁₀) phényl(alcénylène en C₂ à C₁₀), naphtyl(alkylène en C₁ à C₁₀) ou naphtyl(alcénylène en C₂ à C₁₀);
R² est éventuellement substitué par un groupe alkyle ou fluoroalkyle en C₁ à C₄, alcényle en C₂ à C₄, alcoxy ou fluoroalcoxy en C₁ à C₄, des groupes halogènes, -OH, -SH, phényle, phénoxy, -CO₂R³, - N(CH₃)₂, -NO₂ ou -NH₂; cycloalkyle en C₃ à C₁₀, cycloalcényle en C₃ à C₁₀, aryle, (alkyl en C₁ à C₁₀)aryle, un hétérocycle ayant de 3 à 10 chaînons où l'hétéroatome est un ou plusieurs atomes correspondant à N, O, S ou toute combinaison de ceux-ci, ou par toute combinaison de tels substituants,
R² étant éventuellement entrecoupé par un atome d'oxygène, d'azote, de soufre, ou toute combinaison de ceux-ci, et
R³ est, un groupe alkyle en C₁ à C₄, ou alcényle en C₂ à C₄.

9. Composé selon la revendication 8, dans lequel
R¹ est un groupe alcényle en C₂ à C₆, alcynyle en C₂ à C₆, cycloalkyle en C₃ à C₁₀, ou phényle ou naphtyle, substitué par -OH, -SH, ou un groupe alkyle en C₁ à C₄; et
R² est un groupe alkylène en C₁ à C₂₄ ou (alkyl en C₁ à C₁₀)phénylène.

10. Composition comprenant :
(A) un agent biologiquement actif comprenant au moins une protéine, un polypeptide, un peptide, une hormone, un polysaccharide, un mucopolysaccharide, un carbohydrate, ou un lipide; et
(B) un composé ayant la formule 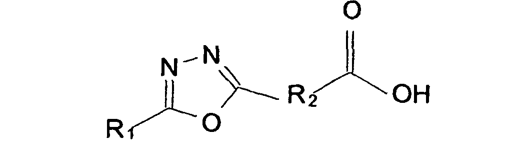
ou un sel de celui-ci, où
R¹ est un groupe alkyle en C₁ à C₁₀, alcényle en C₂ à C₁₀, alcynyle en C₂ à C₁₀, cycloalkyle en C₃ à C₁₀, phényle, naphtyle, ou un hétérocycle aromatique;
R¹ est éventuellement substitué par un groupe alkyle ou fluoroalkyle en C₁ à C₄, alcényle en C₂ à C₄, alcoxy ou fluoroalcoxy en C₁ à C₄, des groupes halogènes, -OH, -SH, un groupe phényle, phénoxy, - CO₂R³, -N(CH₃)₂, -NO₂, ou -NH₂;
R² est un groupe alkylène en C₁ à C₂₄, alcénylène en C₂ à C₂₄, cycloalkylène en C₃ à C₁₀, cycloalcénylène en C₃ à C₁₀, phénylène, naphtylène, (alkyl en C₁ à C₁₀)phénylène, (alcényl en C₁ à C₁₀)phénylène, (alkyl en C₁ à C₁₀)naphtylène, (alcényl en C₂ à C₁₀)naphtylène, phényl(alkylène en C₁ à C₁₀), phényl(alcénylène en C₂ à C₁₀), naphtyl(alkylène en C₁ à C₁₀) ou naphtyl(alcénylène en C₂ à C₁₀);
R² est éventuellement substitué par un groupe alkyle ou fluoroalkyle en C₁ à C₄, alcényle en C₂ à C₄, alcoxy ou fluoroalcoxy en C₁ à C₄, des groupes halogènes, -OH, -SH, phényle, phénoxy, -CO₂R³, - N(CH₃)₂, -NO₂, -NH₂; cycloalkyle en C₃ à C₁₀, cycloalcényle en C₃ à C₁₀, aryle, (alkyl en C₁ à C₁₀)aryle, un hétérocycle ayant de 3 à 10 chaînons où l'hétéroatome est un ou plusieurs atomes correspondant à N, O, S ou toute combinaison de ceux-ci, ou par toute combinaison de tels substituants,
R² étant éventuellement entrecoupé par un atome d'oxygène, d'azote, ou de soufre, ou toute combinaison de ceux-ci, et
R³ est un atome d'hydrogène, un groupe alkyle en C₁ à C₄, ou alcényle en C₂ à C₄.

11. Composition selon la revendication 10, dans laquelle
R¹ est un groupe alkyle en C₁ à C₆, alcényle en C₂ à C₆, alcynyle en C₂ à C₆, cycloalkyle, phényle, ou naphtyle, éventuellement substitué par des groupes halogènes, -OH, -SH, un groupe alkyle en C₁ à C₄, ou alcoxy en C₁ à C₄; et
R² est un groupe alkylène en C₁ à C₂₄ ou (alkyl en C₁ à C₁₀)phénylène.

12. Composition selon la revendication 11, dans laquelle
R¹ est un groupe 2-OH-phényle, éventuellement substitué par -OH, -CI, -CH₃, ou -OCH₃;
R² est un groupe alkyle en C₄ à C₈.

13. Composition comprenant :
(A) un agent biologiquement actif; et
(B) un composé ayant la formule 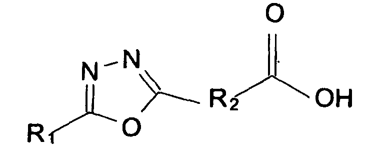
ou un sel de celui-ci, où
R¹ est un groupe cycloalkyle en C₃ à C₁₀ ou naphtyle; éventuellement substitué par un groupe alkyle ou fluoroalkyle en C₁ à C₄, alcényle en C₂ à C₄, alcoxy ou fluoroalcoxy en C₁ à C₄, des groupes halogènes, -OH, -SH, phényle, phénoxy, -CO₂R³, -N(CH₃)₂, -NO₂ ou - NH₂;
R¹ est en outre un groupe 2-hydroxyphényle; éventuellement substitué par -OH, -Cl, -CH₃, ou -OCH₃;
R² est un groupe alkylène en C₁ à C₂₄, alcénylène en C₂ à C₂₄, cycloalkylène en C₃ à C₁₀, cycloalcénylène en C₃ à C₁₀, phénylène, naphtylène, (alkyl en C₁ à C₁₀)phénylène, (alcényl en C₂ à C₁₀)phénylène, (alkyl en C₁ à C₁₀)naphtylène, (alcényl en C₂ à C₁₀)naphtylène, phényl(alkylène en C₁ à C₁₀), phényl(alcénylène en C₂ à C₁₀), naphtyl(alkylène en C₁ à C₁₀) ou naphtyl(alcénylène en C₂ à C₁₀);
R² est éventuellement substitué par un groupe alkyle ou fluoroalkyle en C₁ à C₄, alcényle en C₂ à C₄, alcoxy ou fluoroalcoxy en C₁ à C₄, des groupes halogènes, -OH, -SH, phényle, phénoxy, -CO₂R³, - N(CH₃)₂, -NO₂ ou -NH₂; cycloalkyle en C₃ à C₁₀, cycloalcényle en C₃ à C₁₀, aryle, (alkyl en C₁ à C₁₀)aryle, un hétérocycle ayant de 3 à 10 chaînons où l'hétéroatome est un ou plusieurs atomes correspondant à N, O, S ou toute combinaison de ceux-ci, ou par toute combinaison de tels substituants,
R² étant éventuellement entrecoupé par un atome d'oxygène, d'azote, de soufre, ou toute combinaison de ceux-ci, et
R³ est un atome d'hydrogène, un groupe alkyle en C₁ à C₄, ou alcényle en C₂ à C₄.

14. Composition selon la revendication 13, dans laquelle
R¹ est un groupe cycloalkyle en C₃ à C₁₀ ou naphtyle; éventuellement substitué par des groupes halogènes, -OH, -SH, ou un groupe alkyle en C₁ à C₄ ou alcoxy en C₁ à C₄;
R¹ est en outre un groupe 2-hydroxyphényle; éventuellement substitué par -OH, -CI, -CH₃, ou -OCH₃;
R² est un groupe alkylène en C₁ à C₂₄ ou (alkyl en C₁ à C₁₀)phénylène.

15. Composition selon la revendication 14, dans laquelle
R¹ est un groupe 2-hydroxyphényle; éventuellement substitué par -OH, -Cl, -CH₃, ou -OCH₃;
R² est un groupe alkyle en C₄ à C₈.

16. Composition comprenant
(A) un agent biologiquement actif; et
(B) un composé ayant la formule 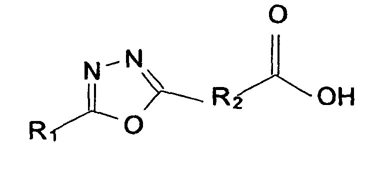
ou un sel de celui-ci, où
R¹ est un groupe alkyle en C₁ à C₁₀, alcényle en C₂ à C₁₀, alcynyle en C₂ à C₁₀, cycloalkyle en C₃ à C₁₀, phényle, naphtyle, ou un hétérocycle aromatique;
R¹ est éventuellement substitué par un groupe alkyle ou fluoroalkyle en C₁ à C₄, alcényle en C₂ à C₄, alcoxy ou fluoroalcoxy en C₁ à C₄, des groupes halogènes, -OH, -SH, phényle, phénoxy, -CO₂R³, -N(CH₃)₂, -NO₂ ou -NH₂;
R² est un groupe alkylène en C₄ à C₈;
R² est éventuellement substitué par un groupe alkyle ou fluoroalkyle en C₁ à C₄, alcényle en C₂ à C₄, alcoxy ou fluoroalcoxy en C₁ à C₄, des groupes halogènes, -OH, -SH, phényle, phénoxy, -CO₂R³,-N(CH₃)₂, -NO₂, -NH₂; cycloalkyle en C₃ à C₁₀, cycloalcényle en C₃ à C₁₀, aryle, (alkyl en C₁ à C₁₀)aryle, un hétérocycle ayant de 3 à 10 chaînons où l'hétéroatome est un ou plusieurs atomes correspondant à N, O, S ou toute combinaison de ceux-ci, ou par toute combinaison de tels substituants,
R² étant éventuellement entrecoupé par un atome d'oxygène, d'azote, de soufre, ou toute combinaison de ceux-ci, et
R³ est un atome d'hydrogène, un groupe alkyle en C₁ à C₄, ou alcényle en C₂ à C₄.

17. Composition selon la revendication 16, dans laquelle
R¹ est un groupe alkyle en C₁ à C₆, alcényle en C₂ à C₆, alcynyle en C₂ à C₆, cycloalkyle, phényle ou naphtyle éventuellement substitué par des groupes halogènes, -OH, -SH, un groupe alkyle en C₁ à C₄, ou alcoxy en C₁ à C₄.

18. Composition selon l'une quelconque des revendications 13 à 17, dans laquelle l'agent biologiquement actif comprend au moins une protéine, un polypeptide, un peptide, une hormone, un polysaccharide, un mucopolysaccharide, un carbohydrate, ou un lipide.

19. Composition selon l'une quelconque des revendications 10 à 18, dans laquelle l'agent biologiquement actif est choisi dans le groupe consistant en les hormones de croissance, les hormones de croissance humaines (HCh), les hormones de croissance humaines recombinantes (HChr), les hormones de croissance bovines, les hormones de croissance porcines, les hormones libératrices de l'hormone de croissance, les interférons, l'interféron-α, l'interféron-β, l'interféron-γ, l'interleukine-1, l'interleukine-2, l'insuline, l'insuline porcine, l'insuline bovine, l'insuline humaine, l'insuline humaine recombinante, le facteur de croissance analogue à l'insuline (IGF), l'IGF-1, l'héparine, l'héparine non fractionnée, les héparinoïdes, les dermatanes, les chondroïtines, l'héparine de bas poids moléculaire, l'héparine de très bas poids moléculaire, l'héparine d'ultra bas poids moléculaire, la calcitonine, la calcitonine de saumon, la calcitonine d'anguille, la calcitonine humaine, l'erythropoïétine (EPO), le facteur natriurétique auriculaire, les antigènes, les anticorps monoclonaux, la somatostatine, les inhibiteurs de protéases, l'adrénocorticotropine, la gonadolibérine, l'oxytocine, l'hormone libératrice de la lutéinostimuline, la folliculostimuline, la glucocérébrosidase, la thrombopoïétine, la filgrastime, les prostaglandines, la cyclosporine, la vasopressine, la cromolyne sodique, le chromoglycate de sodium, le chromoglycate de disodium, la vancomycine, la desferrioxamine (DFO), l'hormone parathyroïdienne (PTH), les fragments de PTH, les agents antimicrobiens, les agents antifongiques, les vitamines, des analogues, des fragments, des agents mimétiques et des dérivés modifiés du polyéthylène-glycol (PEG) de ces composés, et toute combinaison de ceux-ci.

20. Composition selon la revendication 19, dans laquelle l'agent biologiquement actif comprend l'insuline, l'héparine non fractionnée, l'héparine de bas poids moléculaire, l'héparine de très bas poids moléculaire, l'héparine d'ultra bas poids moléculaire, la calcitonine, PTH, EPO, HCh ou toute combinaison de celles-ci.

21. Composition selon la revendication 19, dans laquelle l'agent biologiquement actif comprend la calcitonine de saumon.

22. Composé choisi dans le groupe constitué de 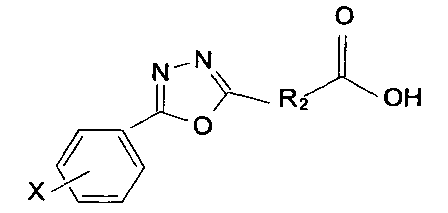
| **Composé #** | **X** | **R**^{**2**} |
|---|---|---|
| | | |
| 1 | 2-OH | (CH₂)₈ |
| 2 | 2-OH | (CH₂)₆ |
| 3 | 2-OH | (CH₂)₄ |
| 4 | 2-OH | (CH₂)₇ |
| 5 | 2-OH-5-Cl | (CH₂)₆ |
| 6 | 2-OH-3,5-di-Cl | (CH₂)₆ |
| 7 | 2-OH-4-méthyl | (CH₂)₆ |
| 8 | 2-O H-4-OMe | (CH₂)₆ |
| 9 | 2-OH-4-OMe | (CH₂)₄ |
| 10 | H | (CH₂)₆ |
| 11 | 2-Cl | (CH₂)₆ |

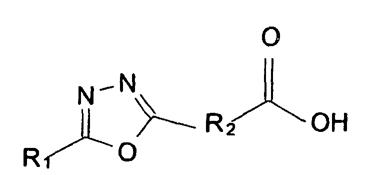
| **Composé #** | **R**^{**1**} | **R**^{**2**} |
|---|---|---|
| | | |
| 12 | CH₃ | (CH₂)₆ |
| 13 | CH₂OH | (CH₂)₆ |
et les ou sels de ceux-ci.

23. Composition comprenant :
(A) un agent actif; et
(B) au moins un composé selon la revendication 22.

24. Composition selon la revendication 23, dans laquelle l'agent actif est choisi dans le groupe consistant en un agent biologiquement actif, un agent chimiquement actif et une combinaison de ces deux.

25. Composition selon la revendication 24, dans laquelle l'agent biologiquement actif comprend au moins une protéine, un polypeptide, un peptide, une hormone, un polysaccharide, un mucopolysaccharide, un carbohydrate, ou un lipide.

26. Composition selon la revendication 24, dans laquelle l'agent biologiquement actif est choisi dans le groupe consistant en les hormones de croissance, les hormones de croissance humaines (HCh), les hormones de croissance humaines recombinantes (HChr), les hormones de croissance bovines, les hormones de croissance porcines, les hormones libératrices de l'hormone de croissance, les interférons, l'interféron-α, l'interféron-β, l'interféron-γ, l'interleukine-1, l'interleukine-2, l'insuline, l'insuline porcine, l'insuline bovine, l'insuline humaine, l'insuline humaine recombinante, le facteur de croissance analogue à l'insuline (IGF), l'IGF-1, l'héparine, l'héparine non fractionnée, les héparinoïdes, les dermatanes, les chondroïtines, l'héparine de bas poids moléculaire, l'héparine de très bas poids moléculaire, l'héparine d'ultra bas poids moléculaire, la calcitonine, la calcitonine de saumon, la calcitonine d'anguille, la calcitonine humaine, l'erythropoïétine (EPO), le facteur natriurétique auriculaire, les antigènes, les anticorps monoclonaux, la somatostatine, les inhibiteurs de protéases, l'adrénocorticotropine, la gonadolibérine, l'oxytocine, l'hormone libératrice de la lutéinostimuline, la folliculostimuline, la glucocérébrosidase, la thrombopoïétine, la filgrastime, les prostaglandines, la cyclosporine, la vasopressine, la cromolyne sodique, le chromoglycate de sodium, le chromoglycate de disodium, la vancomycine, la desferrioxamine (DFO), l'hormone parathyroïdienne (PTH), les fragments de PTH, les agents antimicrobiens, les agents antifongiques, les vitamines, des analogues, des fragments, des agents mimétiques et des dérivés modifiés du polyéthylène-glycol (PEG) de ces composés, et toute combinaison de ceux-ci.

27. Composition selon la revendication 24, dans laquelle l'agent biologiquement actif comprend l'insuline, l'héparine non fractionnée, l'héparine de bas poids moléculaire, l'héparine de très bas poids moléculaire, l'héparine d'ultra bas poids moléculaire, la calcitonine, PTH, EPO, HCh, ou des combinaisons de celles-ci.

28. Composition selon la revendication 24, dans laquelle l'agent biologiquement actif comprend la calcitonine de saumon.

29. Composition selon la revendication 23, dans laquelle le composé est 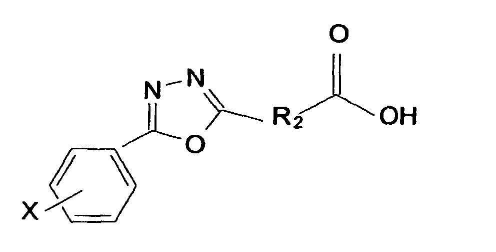 où
| **Composé #** | **X** | **R**^{**2**} |
|---|---|---|
| | | |
| 3 | 2-OH | (CH₂)₄ |
ou un sel de celui-ci.

30. Unité posologique comprenant :
(A) la composition selon la revendication 23; et
(B)
(a) un excipient
(b) un diluant
(c) un agent délitant
(d) un agent lubrifiant
(e) un agent plastifiant
(f) un agent colorant
(g) un véhicule de dosage, ou
(h) toute combinaison de ceux-ci.

31. Unité posologique selon la revendication 30, dans laquelle l'agent biologiquement actif comprend au moins une protéine, un polypeptide, un peptide, une hormone, un polysaccharide, un mucopolysaccharide, un carbohydrate, ou un lipide.

32. Unité posologique selon la revendication 30, dans laquelle l'agent biologiquement actif est choisi dans le groupe consistant en les hormones de croissance, les hormones de croissance humaines (HCh), les hormones de croissance humaines recombinantes (HChr), les hormones de croissance bovines, les hormones de croissance porcines, les hormones libératrices de l'hormone de croissance, les interférons, l'interféron-α, l'interféron-β, l'interféron-γ, l'interteukine-1, l'interleukine-2, l'insuline, l'insuline porcine, l'insuline bovine, l'insuline humaine, l'insuline humaine recombinante, le facteur de croissance analogue à l'insuline (IGF), l'IGF-1, l'héparine, l'héparine non fractionnée, les héparinoïdes, les dermatanes, les chondroïtines, l'héparine de bas poids moléculaire, l'héparine de très bas poids moléculaire, l'héparine d'ultra bas poids moléculaire, la calcitonine, la calcitonine de saumon, la calcitonine d'anguille, la calcitonine humaine, l'erythropoïétine (EPO), le facteur natriurétique auriculaire, les antigènes, les anticorps monoclonaux, la somatostatine, les inhibiteurs de protéases, l'adrénocorticotropine, la gonadolibérine, l'oxytocine, l'hormone libératrice de la lutéinostimuline, la folliculostimuline, la glucocérébrosidase, la thrombopoïétine, la filgrastime, les prostaglandines, la cyclosporine, la vasopressine, la cromolyne sodique, le chromoglycate de sodium, le chromoglycate de disodium, la vancomycine, la desferrioxamine (DFO), l'hormone parathyroïdienne (PTH), les fragments de PTH, les agents antimicrobiens, les agents antifongiques, les vitamines, des analogues, des fragments, des agents mimétiques et des dérivés modifiés du polyéthylène-glycol. (PEG) de ces composés, et toute combinaison de ceux-ci.

33. Unité posologique selon la revendication 31, dans laquelle l'agent biologiquement actif comprend l'insuline, l'héparine non fractionnée, l'héparine de bas poids moléculaire, l'héparine de très bas poids moléculaire, l'héparine d'ultra bas poids moléculaire, la calcitonine, PTH, EPO, HCh ou toute combinaison de celles-ci.

34. Forme d'unité posologique selon la revendication 30, dans laquelle l'agent actif comprend la calcitonine de saumon.

35. Forme d'unité posologique selon la revendication 30, dans laquelle la forme d'unité posologique est sous forme d'un comprimé, d'une capsule, de poudre, ou d'un liquide.

36. Utilisation de la composition selon la revendication 24, pour la préparation d'un médicament pour le traitement des maladies.

37. Procédé de préparation d'une composition comprenant le mélange :
a) d'au moins un agent actif
b) du composé selon la revendication 22;
c) le cas échéant, d'un véhicule de dosage.
